# NEW EUROPEAN PATENT SPECIFICATION

(11) **EP 0 278 776 B2**
(45) Date of publication and mention of the opposition decision: **14.04.2004**
(45) Mention of the grant of the patent: 28.05.1997
(21) Application number: 88301190.0
(22) Date of filing: 12.02.1988
(51) Int. Cl.: C12N 15/12, C07K 14/745, C12N 5/10, C12N 1/21

(54) **Methods and deoxyribonucleic acid for the preparation of tissue factor protein**
Verfahren und Desoxyribonukleinsäure zur Herstellung von Gewebefaktor-Protein
Méthodes et acide desoxyribonucléique pour la préparation de la protéine du facteur tissulaire

(30) Priority: 12.02.1987 US 13743; 07.04.1987 US 35409; 05.02.1988 US 152698
(43) Date of publication of application: 17.08.1988
(60) Divisional application: 97200009.5
(73) Proprietor: GENENTECH, INC., South San Francisco California 94080 (US)
(72) Inventor: Lawn, Richard Mark, San Francisco, CA 94116 (US); Wion, Karen Lynne, Millbrae, CA 94030 (US); Vehar, Gorden Allen, San Carlos, CA 94070 (US)
(74) Representative: Miles, John Stephen

(56) References cited:
- EP-A- 0 139 416
- WO-A-88/09817
- CELL, vol. 50, July 3, 1987, Cambridge, MA (US); J.H. MORRISSEY et al., pp. 129-135
- BIOCHEMISTRY, vol. 26, no. 17, 25 August 1987, Washington, DC (US); E.M. SCARPATI et al. pp. 5234-5238
- THROMBOSIS RESEARCH, vol. 48, no. 1, October 1987, New York, NY (US); K.L. FISHER et al., pp. 89-99
- PROCEEDINGS OF THE NATL. ACADEMY OF SCIENCES USA, vol. 84, no. 15, August 1987, Washington, DC (US); E.K. SPICER et al., pp. 5148-5152
- FEDERATION PROCEEDINGS, vol. 45, no. 6, May 1986, Bethesda, MD (US); J.M. KITTLER, p. 1639, no. 927
- THROMBOSIS & HAEMOSTASIS, vol. 58, no. 1, January 1987, Stuttgart (DE); T.S. EDGINGTON et al., p. 258, no. 941
- PROCEEDINGS OF THE NATL. ACADEMY OF SCIENCES USA, vol. 83, January 1986, Biochemistry, Washington, DC (US); A. GUHA et al., pp. 299-302
- FEDERATION PROCEEDINGS, vol. 46, no. 3, 01 March 1987, Bethesda, MD (US);: J.H. MORRISSEY et al., p. 716, no. 2338
- FEDERATION PROCEEDINGS, vol. 46, no. 6, 01 May 1987, Bethesda, MD (US); E.M. SCARPATI ET AL: page 2242

## Description

This invention relates to preparation of isolated deoxyribonucleic acid (DNA) coding for the production of tissue factor protein, to methods of obtaining DNA molecules which code for tissue factor protein, to the expression of human tissue factor protein utilizing such DNA, as well as to novel compounds, including novel nucleic acids encoding tissue factor protein or fragments thereof. This invention is also directed to tissue factor protein derivatives, particularly derivatives lacking the near C-terminal cytoplasmic and/or hydrophobic portion of the protein, and their production by recombinant DNA techniques.

Bleeding is one of the most serious and significant manifestations of disease. It may occur from a local site or may be generalized. Primary hemostasis consists principally of two components: vasoconstriction and platelet plug formation. Platelet plug formation may be divided into several stages: adhesion of platelets to subendothelial surfaces exposed by trauma; platelet activation release reaction; platelet aggregation, which results in the sequestration of additional platelets at the site, and the binding of fibrinogen and the coagulation proteins to the platelet surface which results in thrombin formation: and, fusion which is the coalescence of fibrin and fused platelets to form a stable haemostatic plug.

Blood coagulation performs two functions; the production of thrombin which induces platelet aggregation and the formation of fibrin which renders the platelet plug stable. A number of discrete proenzymes and procofactors, referred to as "coagulation factors", participate in the coagulation process. The process consists of several stages and ends with fibrin formation. Fibrinogen is converted to fibrin by the action of thrombin. Thrombin is formed by limited proteolysis of a proenzyme, prothrombin. This proteolysis is effected by activated factor X (referred to as factor Xₐ) which binds to the surface of activated platelets and, in the presence of factor Va and ionic calcium, cleaves prothrombin.

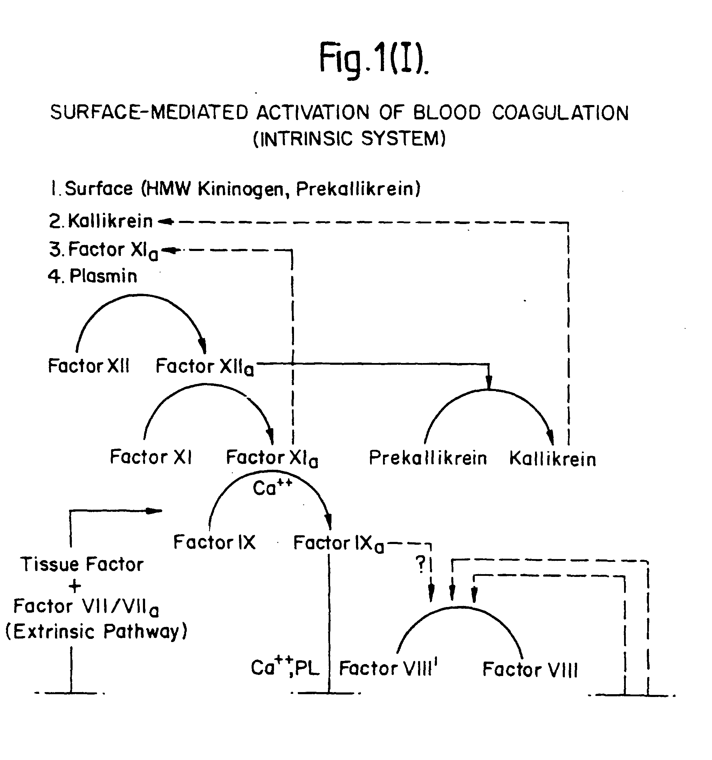

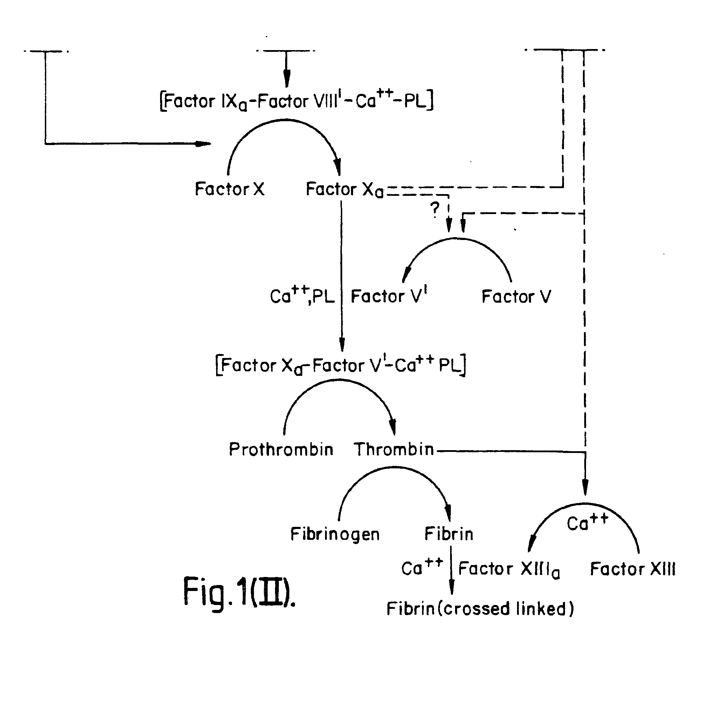

Activation of factor X may occur by either of two separate pathways, the extrinsic or the intrinsic (Figure 1). The intrinsic cascade consists of a series of reactions wherein a protein precursor is cleaved to form an active protease. At each step, the newly formed protease will catalyze the activation of the precursor protease at the subsequent step of the cascade. A deficiency of any of the proteins in the pathway blocks the activation process at that step, thereby preventing clot formation and typically gives rise to a tendency to hemorrhage. Deficiencies of factor VIII or factor IX, for example, cause the severe bleeding syndromes haemophilia A and B, respectively. In the extrinsic pathway of blood coagulation, tissue factor, also referred to as tissue thromboplastin, is released from damaged cells and activates factor X in the presence of factor VII and calcium. Although activation of factor X was originally believed to be the only reaction catalyzed by tissue factor and factor VII, it is now known that an amplification loop exists between factor X, factor VII, and factor IX (Osterud, B., and S.I. Rapaport, Proc. Natl. Acad. Sci. [USA] 74:5260-5264 [1977]; Zur, M. et al., Blood 52: 198 [1978]). Each of the serine proteases in this scheme is capable of converting by proteolysis the other two into the activated form, thereby amplifying the signal at this stage in the coagulation process (Figure 1). It is now believed that the extrinsic pathway may in fact be the major physiological pathway of normal blood coagulation (Haemostasis 13:150-155 [1983]). Since tissue factor is not normally found in the blood, the system does not continuously clot; the trigger for coagulation would therefore be the release of tissue factor from damaged tissue.

Tissue factor is believed to be an integral membrane glycoprotein which, as discussed above, can trigger blood coagulation via the extrinsic pathway (Bach, R. et al., J. Biol Chem. 256[16]: 8324-8331 [1981]). Tissue factor consists of a protein component (previously referred to as tissue factor apoprotein-III) and a phospholipid. Osterud, B. and Rapaport, S.I., Proc.Natl.Acad.Sci. 74, 5260-5264 (1977). The complex has been found on the membranes of monocytes and different cells of the blood vessel wall (Osterud, B., Scand. J. Haematol. 32: 337-345 [1984]). Tissue factor from various organs and species has been reported to have a relative molecular mass of 42,000 to 53,000. Human tissue thromboplastin has been described as consisting of a tissue factor protein inserted into a phospholipid bilayer in an optimal ratio of tissue factor protein:phospholipid of approximately 1:80 (Lyberg, T. and Prydz, H., Nouv. Rev. Fr. Hematol. 25(5): 291-293 [1983]). Purification of tissue factor has been reported from various tissues such as,: human brain (Guha, A. et al. Proc. Natl. Acad. Sci. 83: 299-302 [1986] and Broze,G.H. et al., J.Biol.Chem. 260[20]: 10917-10920 [1985]); bovine brain (Bach, R. et al., J. Biol. Chem. 256: 8324-8331 [1981]); human placenta (Bom, V J.J. et al., Thrombosis Res. 42:635-643 [1986]; and, Andoh, K. et al., Thrombosis Res. 43:275-286 [1986]); ovine brain (Carlsen E. et al., Thromb. Haemostas. 48[3], 315-319 [1982]); and, lung (Glas, P. and Astrup, T, Am. J. Physiol. 219, 1140-1146 [1970]). It has been shown that bovine and human tissue thromboplastin are identical in size and function (see Broze, G.H. et al., J. Biol. Chem. 260[20], 10917-10920 [1985]). It is widely accepted that while there are differences in structure of tissue factor protein between species there are no functional differences as measured by in vitro coagulation assays (Guha et al. supra). Furthermore, tissue factor isolated from various tissues of an animal, e.g. dog brain, lung, arteries and vein was similar in certain respects such as, extinction coefficient, content of nitrogen and phosphorous and optimum phospholipid to lipid ratio but differed slightly in molecular size, amino acid content, reactivity with antibody and plasma half life (Gonmori, H. and Takeda, Y., J. Physiol. 229[3], 618-626 [1975]). All of the tissue factors from the various dog organs showed clotting activity in the presence of lipid. Id. It is widely accepted that in order to demonstrate biological activity, tissue factor must be associated with phospholipids in vitro (Pitlick, F.A., and Nemerson, Y., Biochemistry 9: 5105-5111 [1970] and Bach,R. et al. supra. at 8324). It has been shown that the removal of the phospholipid component of tissue factor, for example by use of a phospholipase, results in a loss of its biological activity in vitro (Nemerson, Y., J.C.I. 47: 72-80 [1968]). Relipidation can restore in vitro tissue factor activity (Pitlick, F. A. and Nemerson, Y., supra and Freyssinet, J.M. et al., Thrombosis and Haemostasis 55: 112-118 [1986]). Amino terminal sequences of tissue factor (Bach, R. et al., Am. Heart Assoc. [Nov., 1986], Morrissey, J.H. et al., Am. Heart Assoc. [Nov., 1986]) and a CNBr peptide fragment (Bach, R. et al. supra) have been determined.

Infusion of tissue factor has long been believed to compromise normal haemostasis. In 1834 the French physiologist de Blainville first established that tissue factor contributed directly to blood coagulation (de Blainville, H. Gazette Medicale Paris, Series 2, 524 [1834]). de Blainville also observed that intravenous infusion of a brain tissue suspension caused immediate death which he observed was correlated with a hypercoagulative state giving rise to extensively disseminated blood clots found on autopsy. It is now well accepted that intravenous infusion of tissue thromboplastin induces intravascular coagulation and may cause death in various animals (Dogs: Lewis, J. and Szeto I.F., J. Lab. Clin. Med 60: 261-273 [1962]: rabbits: Fedder, G. et al., Thromb. Diath. Haemorrh. 27: 365-376 [1972]; rats: Giercksky, K. E. et al., Scand. J. Haematol. 17: 305-311 [1976]: and, sheep: Carlsen,E. et al., Thromb. Haemostas. 48: 315-319 [1982]).

Although the isolation of tissue factor has been described in the literature as shown above, the precise structure of tissue factor protein has not been previously established. While some quantities of "purified" tissue factor protein have been available as obtained from various tissues, the low concentration of tissue factor protein in blood and tissues and the high cost, both economic and of effort, of purifying the protein from tissues makes this a scarce material. It is an object of the present invention to isolate DNA encoding tissue factor protein and to produce useful quantities of human tissue factor protein using recombinant techniques. It is a further object to prepare novel forms of tissue factor protein. This and other objects of this invention will be apparent from the specification as a whole.

Objects of this invention can be accomplished by a method comprising: identifying and cloning the cDNA which codes for human tissue factor protein ; incorporating that cDNA into a recombinant DNA vector; transforming a suitable host with the vector including that DNA; expressing the human tissue factor protein DNA in such a host; and recovering the human tissue factor protein that is produced. Similarly, the present invention makes it possible to produce human tissue factor protein and/or derivatives thereof by recombinant techniques, as well as to provide products and methods related to such human tissue factor protein production. The isolation and identification and sequencing of the tissue factor protein DNA was problematic. The mRNA was relatively rare and heretofore no complete amino acid sequence for tissue factor protein was known.

The present invention is directed to the compositions and methods of producing human tissue factor protein via recombinant DNA technology, including: 1) the isolation and identification of the entire DNA sequence of the protein and the 5' and 3'-flanking region thereof; 2) the construction of cloning and expression vehicles comprising said DNA sequence, enabling the expression of the human tissue factor protein, as well as methionine, fusion or signal N-terminus conjugates thereof: and 3) viable cell cultures, genetically altered by virtue of their containing such vehicles and capable of producing human tissue factor protein. This invention is further directed to DNA compositions and methods of producing DNA which codes for cellular production of human tissue factor protein. Yet another aspect of this invention are new compounds, including DNA sequences which are utilized in obtaining clones which encode tissue factor protein. This invention is further directed to novel tissue factor protein derivatives, in particular derivatives lacking the signal sequence and the hydrophobic portion of the protein near the C-terminal end of the protein comprising the amino acid sequence which constitutes the tissue factor protein transmembrane or membrane binding domain.

The utility of the human tissue factor protein and derivatives thereof of this invention is based in part on the novel and unexpected observation that infusion into hemophilic dogs of tissue factor protein, that is the protein portion of tissue factor lacking the naturally occurring phospholipid, which was previously referred to as tissue factor apoprotein III and previously believed to be inactive, corrected the haemostatic deficiency. Tissue factor protein was for the first time found to correct the bleeding diathesis, i.e. a tendency toward hemorrhage, associated with factor VIII deficiency in vivo. Infusion of tissue factor protein would be expected to be ineffective in light of the prior art papers which describe tissue factor as having an absolute requirement for phospholipid. In contrast to the work of de Blainville and subsequent researchers over the next one hundred and fifty-two (152) years, tissue factor protein was also found to be nontoxic to the dogs when infused intravenously.

The human tissue factor protein and derivatives thereof of this invention are useful in the treatment of various chronic bleeding disorders, characterized by a tendency toward hemorrhage, both inherited and acquired. Examples of such chronic bleeding disorders are deficiencies of factors VIII, IX, or XI. Examples of acquired disorders include: acquired inhibitors to blood coagulation factors e.g. factor VIII, von Willebrand factor, factors IX, V, XI, XII and XIII; haemostatic disorder as a consequence of liver disease which includes decreased synthesis of coagulation factors and DIC; bleeding tendency associated with acute and chronic renal disease which includes coagulation factor deficiencies and DIC: haemostasis after trauma or surgery: patients with disseminated malignancy which manifests in DIC with increases in factors VIII, von Willebrand factor and fibrinogen; and haemostasis during cardiopulmonary surgery and massive blood transfusion. The human tissue factor protein and derivatives thereof of this invention may also be used to induce coagulation for acute bleeding problems in normal patients and in those with chronic bleeding disorders. Other uses for tissue factor protein will be apparent to those skilled in the art.

### Brief Description of the Drawings

- Fig. 1: Diagram showing activation of blood coagulation via intrinsic pathway.
- Fig. 2: Nucleotide and amino acid sequence of human tissue factor protein. The nucleotide sequence of the human tissue factor protein was determined from DNA sequence analysis of one adipose clone and in part confirmed by sequencing other clones. Predicted amino acids of the tissue factor protein are shown below the DNA sequence and are numbered from the first residue of the N-terminal of the protein sequence. Negative amino acid numbers refer to the presumed leader signal sequence or preprotein, while positive numbers refer to the mature protein.

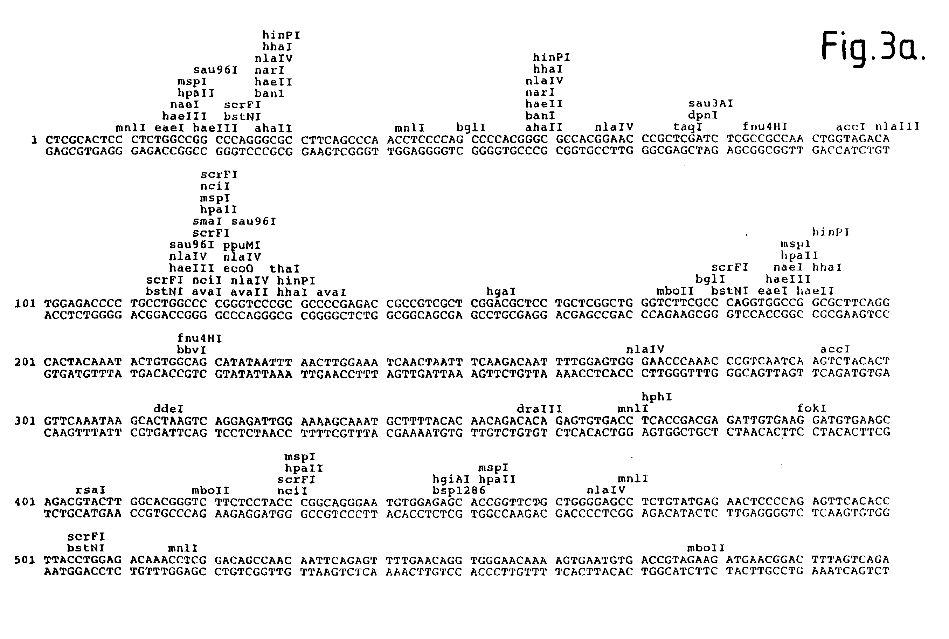

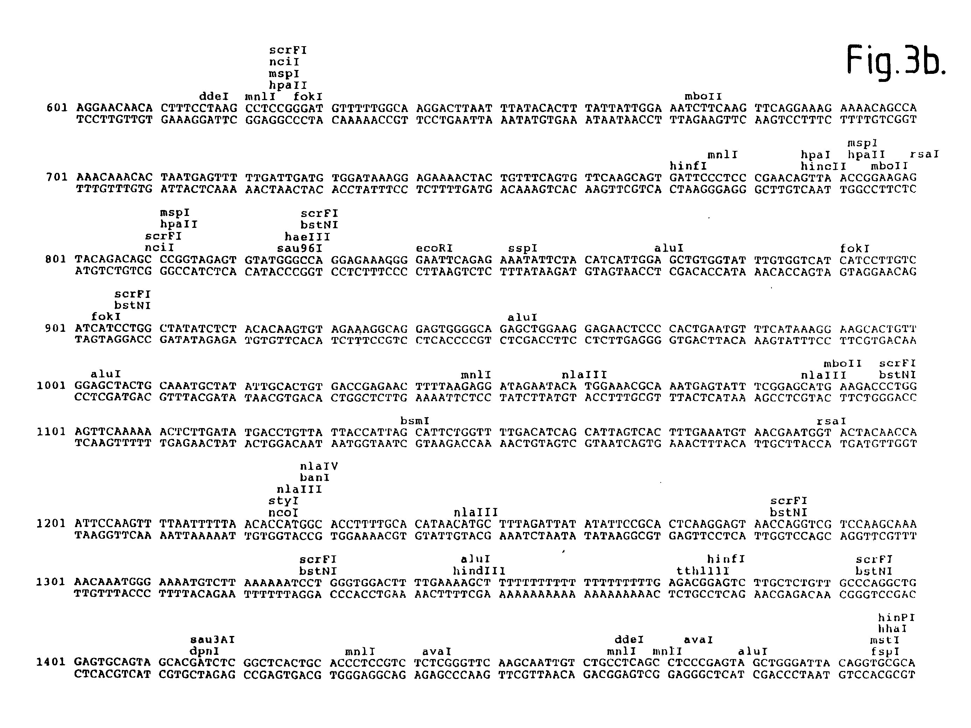

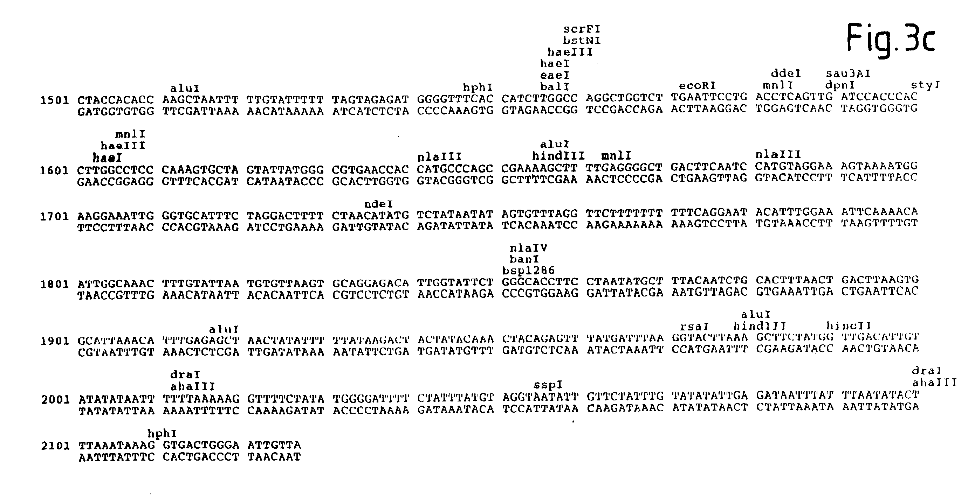

- Figs. 3a-3c: (Collectively referred to herein as Fig. 3). The human tissue factor protein cDNA and restriction enzyme sites.
- Fig. 4: Construction of an expression vector pCISTF encoding full length tissue factor protein for use in mammalian host cells.
- Fig. 4a: Illustrates part of the construction of pCIS2.8c series vectors used herein.

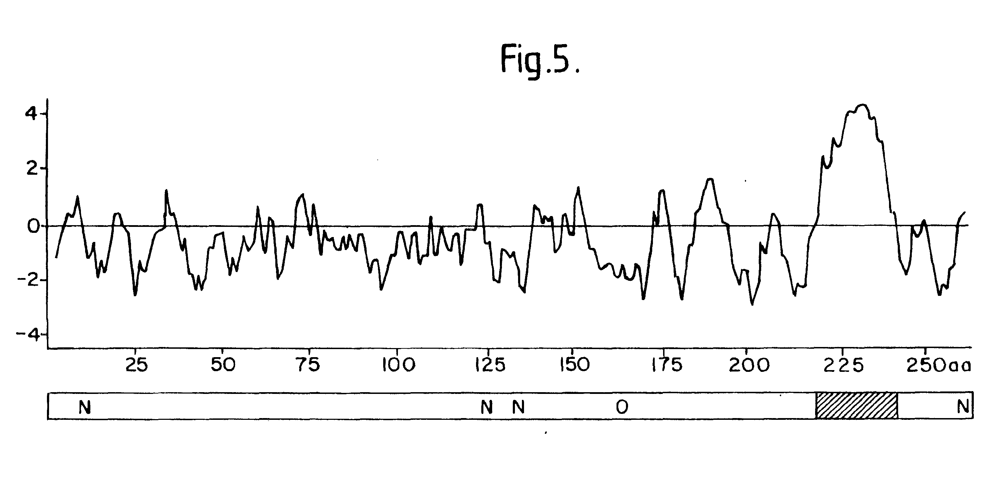

- Fig.5: Hydropathy profile of tissue factor. The translated DNA sequence of human tissue factor was plotted using the algorithm of Kyte and Doolittle, J. Mol. Biol., 157: 105 (1982). The abscissa shows the amino acid sequence beginning at the mature amino terminus. Positive points in the ordinate denote hydrophobic regions of the protein; each point represents the average hydropathy of 6 successive amino acids. At the bottom, N marks the location of predicted asparagine-linked glycosylation sites and 0 marks the cluster of serine and threonine residues at amino acids 160-172. The predicted hydrophobic membrane spanning domain encompasses residues 220-243 and is indicated by a filled bar.
- Fig. 6: Construction of an expression vector pTF 2A12 encoding full length tissue factor protein.
- Figs. 7a-c: Figs. 7a-c are collectively referred to herein as Fig. 7. Construction of tissue factor protein mutant having a serine substituted for a cysteine at position 245.

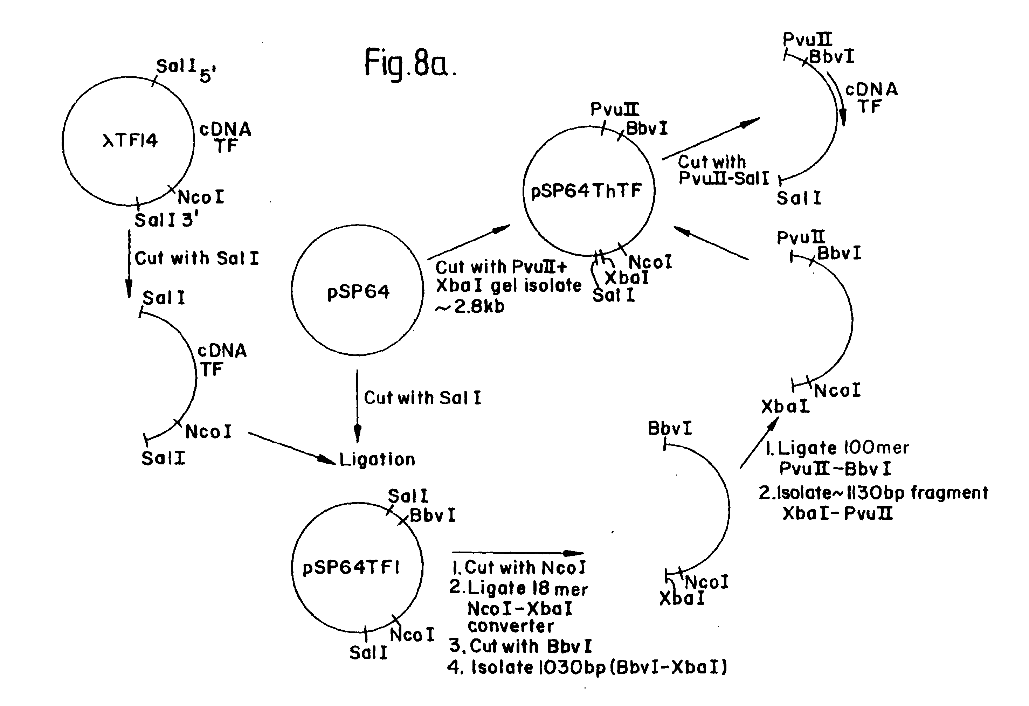

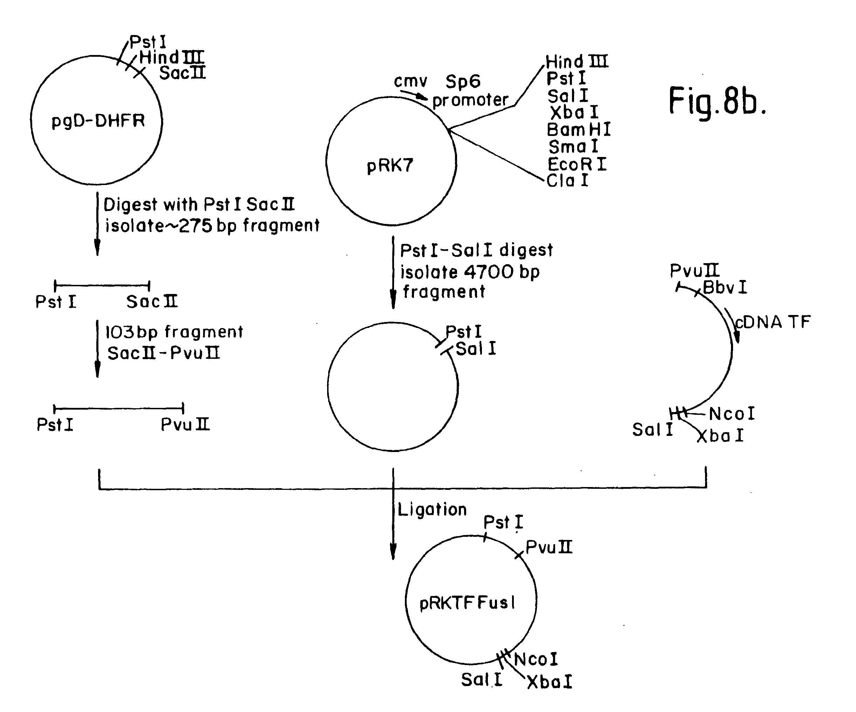

- Figs.8a-b: Figs. 8a-8b are collectively referred to herein as Figure 8. Construction of an expression vector for human tissue factor fusion protein.
- Fig. 9: Chromogenic assay results of transient expression of tissue factor protein in Cos cells.
- Fig. 10: Construction of an expression vector for cytoplasmic domain deleted tissue factor protein.

### Detailed Description

As used herein, "tissue factor protein" refers to a protein capable of correcting various bleeding disorders e.g. by inducing coagulation, particularly those disorders associated with deficiencies in coagulation factors. Tissue factor protein is distinct from tissue factor or tissue thromboplastin of the prior art in that it lacks the naturally occurring lipid portion of the molecule. Tissue factor protein also includes tissue factor protein associated with phospholipid which lipid is distinct from the naturally occurring lipid associated with tissue thromboplastin and displays coagulation-inducing capability without the concomitant toxicity observed with the lipidated protein. Infusion of tissue factor protein, as defined herein, does not result in disseminated intravascular coagulation. The capacity of tissue factor protein to correct various bleeding disorders is readily determined using various in vivo bleeding models e.g. initiation of coagulation in hemophilic dogs using cuticle bleeding time (CBT) determination (Giles, A.R. et al., Blood 60:727-730 [1982]).

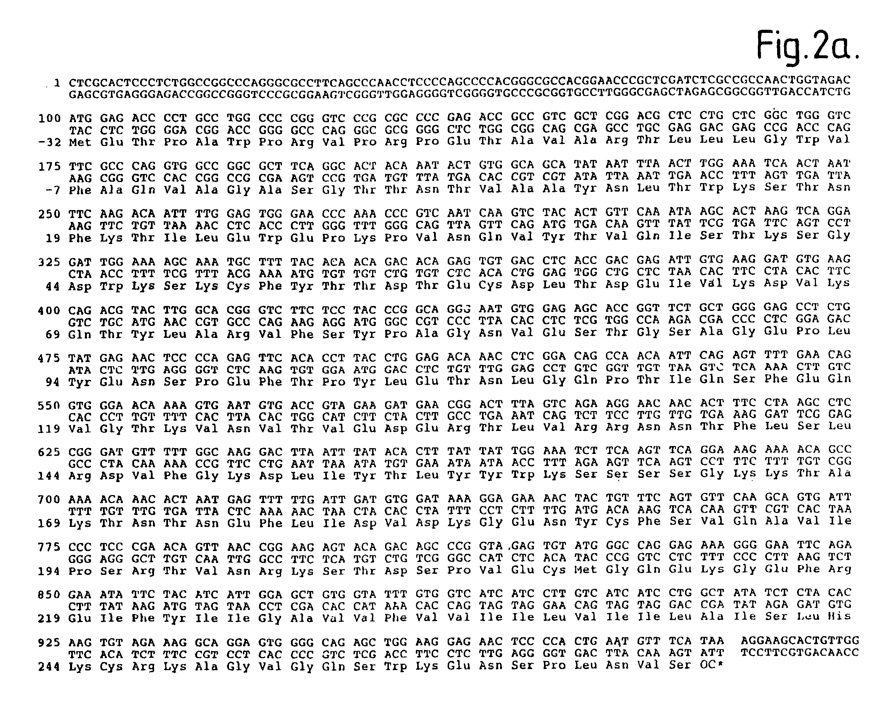

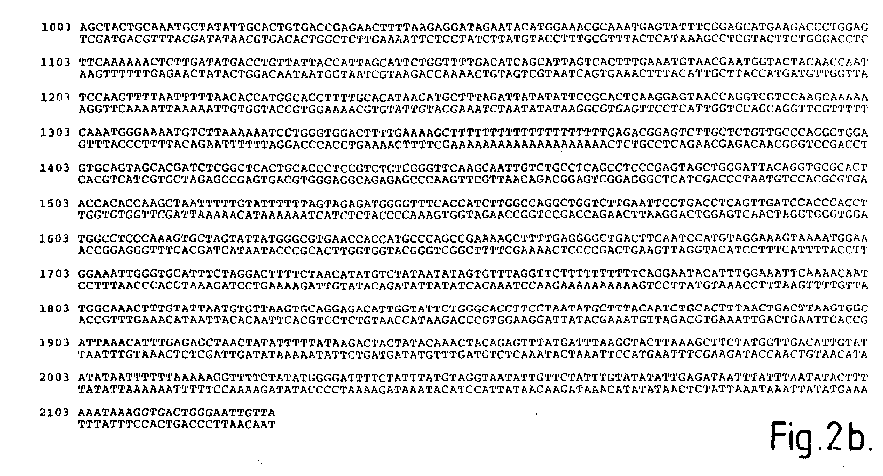

The amino acid sequence of figure 2 is that of pre-tissue factor protein. Pre-tissue factor protein can be expressed, for example, in prokaryotes, which do not process and secrete mature protein, by transforming with an expression vector comprising DNA encoding pre-tissue factor protein. It is preferable to transform host cells capable of accomplishing such processing so as to obtain mature tissue factor protein in the culture medium or periplasm of the host cell. Typically, higher eukaryotic host cells such as mammalian cells are capable of processing pre-tissue factor protein and secreting mature tissue factor protein upon transformation with DNA encoding pre-tissue factor protein.

Alternatively, secreted mature tissue factor protein can be obtained by ligating the 5' end of the DNA encoding mature tissue factor protein to the 3' end of DNA encoding a signal sequence recognized by the host cell. An expression vector comprising the ligated DNA sequences is used to transform host cells. The host cell will process the expressed fusion by proteolytically cleaving the peptide bond between the signal sequence and the first amino acid of tissue factor protein and secreting the mature tissue factor protein into the host cell periplasm or into the medium, depending upon the host cell in question. For example, in constructing a prokaryotic expression vector the human tissue factor protein secretory leader, i.e. amino acids -32 to -1, is replaced by the bacterial alkaline phosphatase or heat stable enterotoxin II leaders, and for yeast the tissue factor protein leader is replaced by the yeast invertase, alpha factor or acid phosphatase leaders. Gram negative organisms transformed with a homologous signal-tissue factor protein fusion will secrete mature tissue factor protein into the cell periplasm, whereas yeast or bacillus sp. will secrete mature tissue factor protein into the culture medium.

Included within the scope of the present invention are, deglycosylated or unglycosylated derivatives of such tissue factor proteins, and biologically active amino acid sequence variants of tissue factor protein, including alleles, and in vitro-generated covalent derivatives of tissue factor proteins that demonstrate tissue factor protein activity.

Amino acid sequence variants of tissue factor protein fall into one or more of three classes: substitutional, insertional or deletional variants. Insertions include amino and/or carboxyl terminal fusions as well as intrasequence insertions of single or multiple amino acid residues. Tissue factor fusion proteins include, for example, hybrids of mature tissue factor protein with polypeptides that are homologous with tissue factor protein, for example, in the case of human tissue factor protein, secretory leaders from other secreted human proteins. Tissue factor fusion proteins also include hybrids of tissue factor protein with polypeptides horologous to the host cell but not to tissue factor protein, as well as, polypeptides heterologous to both the host cell and the tissue factor protein. An example of such tissue factor fusion protein is the herpes gD-signal sequence with the mature tissue factor protein. Preferred fusions within the scope of this invention are amino terminal fusions with either prokaryotic peptides or signal peptides of prokaryotic, yeast, viral or host cell signal sequences. It is not essential that the signal sequence be devoid of any residual mature sequence from the protein whose secretion it ordinarily directs but this is preferable in order to avoid the secretion of a tissue factor protein fusion.

Insertions can also be introduced within the mature coding sequence of tissue factor protein. These, however, ordinarily will be smaller insertions than those of amino or carboxyl terminal fusions, on the order of 1 to 4 residues. A representative example is [Arg₁₃₅Arg₁₃₆→Arg₁₃₅ProArg₁₃₇] tissue factor protein, a variant selected for its resistance to trypsin hydrolysis at the Arg₁₃₅ residue. Unless otherwise stated, the specific tissue factor protein variations described herein are variations in the mature tissue factor protein sequence; they are not pre-tissue factor protein variants.

Insertional amino acid sequence variants of tissue factor proteins are those in which one or more amino acid residues are introduced into a predetermined site in the target tissue factor protein. Most commonly, insertional variants are fusions of heterologous proteins or polypeptides to the amino or carboxyl terminus of tissue factor protein. Immunogenic tissue factor protein derivatives are made by fusing a polypeptide sufficiently large to confer immunogenicity to the target sequence by crosslinking in vitro or by recombinant cell culture transformed with DNA encoding the fusion. Such immunogenic polypeptides can be bacterial polypeptides such as trpLE, beta-galactosidase and the like.

Deletion variants are characterized by the removal of one or more amino acid residues from the tissue factor protein sequence. Typically, no more than about from 2 to 6 residues are deleted at any one site within the tissue factor protein molecule, although deletion of residues -31 to -1 inclusive will be undertaken to obtain met-tissue factor protein, a variant adapted for intracellular direct expression of met-mature tissue factor protein. Another deletion variant is of the transmembrane domain located at about residues 220 to 242 of the tissue factor protein molecule.

These variants ordinarily are prepared by site specific mutagenesis of nucleotides in the DNA encoding the tissue factor protein, thereby producing DNA encoding the variant, and thereafter expressing the DNA in recombinant cell culture. However, variant tissue factor protein fragments having up to about 100-150 residues may be conveniently prepared by in vitro synthesis. The variants typically exhibit the same qualitative biological activity as the naturally-occurring analogue, although variants also are selected in order to modify the characteristics of tissue factor protein as will be more fully described below.

While the site for introducing an amino acid sequence variation is predetermined, the mutation per se need not be predetermined. For example, in order to optimize the performance of a mutation at a given site, random mutagenesis may be conducted at the target codon or region and the expressed tissue factor protein variants screened for the optimal combination of desired activity. Techniques for making substitution mutations at predetermined sites in DNA having a known sequence are well known, for example M13 primer mutagenesis.

Amino acid substitutions are typically of single residues; insertions usually will be in the order of about from 1 to 10 amino acid residues; and deletions will range about from 1 to 30 residues. Deletions or insertions preferably are made in adjacent pairs, i.e. a deletion of 2 residues or insertion of 2 residues. Substitutions, deletions, insertions or any combination thereof may be combined to arrive at a final construct. Obviously, the mutations that will be made in the DNA encoding the variant tissue factor protein must not place the sequence out of reading frame and preferably will not create complementary regions that could produce secondary mRNA structure (EP 75,444A).

Substitutional variants are those in which at least one residue in the Fig. 2 sequence has been removed and a different residue inserted in its place. Such substitutions generally are made in accordance with the following Table 1 when it is desired to finely modulate the characteristics of tissue factor protein.

**TABLE 1**

| Original Residue | Exemplary Substitutions |
|---|---|
| Ala | ser |
| Arg | lys |
| Asn | gln: his |
| Asp | glu |
| Cys | ser |
| Gln | asn |
| Glu | asp |
| Gly | pro |
| His | asn; gln |
| Ile | leu; val |
| Leu | ile; val |
| Lys | arg; gln; glu |
| Met | leu; ile |
| Phe | met; leu; tyr |
| Ser | thr |
| Thr | ser |
| Trp | tyr |
| Tyr | trp; phe |
| Val | ile; leu |

Substantial changes in function or immunological identity are made by selecting substitutions that are less conservative than those in Table 1, i.e., selecting residues that differ more significantly in their effect on maintaining (a) the structure of the polypeptide backbone in the area of the substitution, for example as a sheet or helical conformation, (b) the charge or hydrophobicity of the molecule at the target site or (c) the bulk of the side chain. The substitutions which in general are expected to produce the greatest changes in tissue factor protein properties will be those in which (a) a hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine.

A major class of substitutional or deletional variants are those involving the transmembrane, i.e. hydrophobic or lipophilic, region of tissue factor protein. The transmembrane region of tissue factor protein is located at about residues 220 to 242 of the protein encoded by the DNA from human adipose tissues. This region is a highly hydrophobic or lipophilic domain that is the proper size to span the lipid bilayer of the cellular membrane. It is believed to anchor tissue factor protein in the cell membrane.

Deletion or substitution of the transmembrane domains will facilitate recovery and provide a soluble form of recombinant tissue factor protein by reducing its cellular or membrane lipid affinity and improving its water solubility so that detergents will not be required to maintain tissue factor protein in aqueous solution. Preferably, the transmembrane domain is deleted, rather than substituted in order to avoid the introduction of potentially immunogenic epitopes. One advantage of the transmembrane deleted tissue factor protein is that it is more easily secreted into the culture medium. This variant is water soluble and does not have an appreciable affinity for cell membrane lipids, thus considerably simplifying its recovery from recombinant cell culture.

Substitutional or deletional mutagenesis can be employed to eliminate N- or O-linked glycosylation sites (e.g. by deletion or substitution of asparaginyl residues in Asn-X-Thr glycosylation sites), improve expression of tissue factor protein or alter the half life of the protein. Alternatively, unglycosylated tissue factor protein can be produced in recombinant prokaryotic cell culture. Deletions or substitutions of cysteine or other labile residues also may be desirable, for example in increasing the oxidative stability or selecting the preferred disulfide bond arrangement of the tissue factor protein. One such example of a cysteine substitution is the substitution of a serine for the cysteine at position 245. Deletions or substitutions of potential proteolysis sites, e.g. Arg Arg, is accomplished for example by deleting one of the basic residues or substituting one by glutaminyl or histidyl residues.

A DNA isolate is understood to mean chemically synthesized DNA, cDNA or genomic DNA with or without the 3' and/or 5' flanking regions. DNA encoding tissue factor protein is obtained from other sources than human by a) obtaining a cDNA library from the placenta, adipose or other tissues containing tissue factor protein mRNA, such as brain, of the particular animal, b) conducting hybridization analysis with labelled DNA encoding human tissue factor protein or fragments thereof (usually, greater than 100bp) in order to detect clones in the cDNA library containing homologous sequences, and c) analyzing the clones by restriction enzyme analysis and nucleic acid sequencing to identify full-length clones. If full length clones are not present in the library, then appropriate fragments may be recovered from the various clones using nucleic acid sequence disclosed for the first time in the present invention and ligated at restriction sites common to the clones to assemble a full-length clone encoding tissue factor protein.

Covalent modifications of the tissue factor protein molecule are included within the scope of the invention. Such modifications are made by reacting targeted amino acid residues of the recovered protein with an organic derivatizing agent that is capable of reacting with selected side chains or terminal residues. Alternately, post-translational modification in selected recombinant host cells may be used to modify the protein. The resulting covalent derivatives are useful as immunogens or to identify residues important for biological activity as well as for altering pharmacological characteristics of the molecule, such as half life, binding affinity and the like, as would be known to the ordinarily skilled artisan.

Certain post-translational derivatizations are the result of the action of recombinant host cells on the expressed polypeptide. Glutaminyl and asparaginyl residues are frequently post-translationally deamidated to the corresponding glutamyl and aspartyl residues. Alternatively, these residues are deamidated under mildly acidic conditions. Either form of these residues falls within the scope of this invention.

Other post-translational modifications include hydroxylation of proline and lysine, phosphorylation of hydroxyl groups of seryl or threonyl residues, methylation of the α-amino groups of lysine, arginine, and histidine side chains (T.E. Creighton, Proteins: Structure and Molecular Properties, W.H. Freeman & Co., San Francisco pp 79-86 [1983]), acetylation of the N-terminal amine and, in some instances, amidation of the C-terminal carboxyl.

"Essentially free from" or "essentially pure" when used to describe the state of tissue factor protein produced by the invention means free of protein or other materials normally associated with tissue factor protein in its in vivo physiological milieu as for example when tissue factor protein is obtained from blood and/or tissues by extraction and purification. Other materials include infectious organisms such as, for example, the causative agent of acquired deficiency syndrome (AIDS) Tissue factor protein produced by the method of the instant invention is greater than or equal to 95% purity.

In general, prokaryotes are used for cloning of DNA sequences in constructing the vectors useful in the invention. For example, E. coli K12 strain 294 (ATCC No. 31446) is particularly useful. Other microbial strains which may be used include E. coli B and E. coli X1776 (ATCC No. 31537) These examples are illustrative rather than limiting.

Prokaryotes also can be used for expression. The aforementioned strains, as well as E coli W3110 (F⁻, λ⁻, prototrophic, ATTC No. 27325), bacilli such as Bacillus subtilus, and other enterobacteriaceae such as Salmonella typhimurium or Serratia marcescans. and various pseudomonas species can be used.

In general, plasmid vectors containing promoters and control sequences which are derived from species compatible with the host cell are used with these hosts. The vector ordinarily carries a replication site as well as one or more marker sequences which are capable of providing phenotypic selection in transformed cells. For example. E. coli is typically transformed using a derivative of pBR322 which is a plasmid derived from an E. coli species (Bolivar, et al., Gene 2: 95 [1977]) pBR322 contains genes for ampicillin and tetracycline resistance and thus provides easy means for identifying transformed cells. The pBR322 plasmid, or other microbial plasmid must also contain or be modified to contain promoters and other control elements commonly used in recombinant DNA construction.

Promoters suitable for use with prokaryotic hosts illustratively include the β-lactamase and lactose promoter systems (Chang et al., "Nature", 275: 615 [1978]; and Goeddel et al., "Nature" 281: 544 [1979]), alkaline phosphatase, the tryptophan (trp) promoter system (Goeddel "Nucleic Acids Res." 8: 4057 [1980] and EPO Appln. Publ. No. 36,776) and hybrid promoters such as the tac promoter (H. de Boer et al., "Proc. Natl. Acad. Sci. USA" 80: 21-25 [1983]). However, other functional bacterial promoters are suitable. Their nucleotide sequences are generally known, thereby enabling a skilled worker operably to ligate them to DNA encoding tissue factor protein using linkers or adaptors to supply any required restriction sites (Siebenlist et al., "Cell" 20: 269 [1980]). Promoters for use in bacterial systems also will contain a Shine-Dalgarno (S.D.) sequence operably linked to the DNA encoding tissue factor protein.

In addition to prokaryotes, eukaryotic microbes such as yeast cultures may also be used. Saccharomyces cerevisiae, or common baker's yeast is the most commonly used eukaryotic microorganism, although a number of other strains are commonly available. For expression in Saccharomyces, the plasmid YRp7, for example, (Stinchcomb, et al., Nature 282: 39 [1979]; Kingsman et al, Gene 7: 141 [1979]; Tschemper et al., Gene 10: 157 [1980]) is commonly used. This plasmid already contains the trpl gene which provides a selection marker for a mutant strain of yeast lacking the ability to grow in tryptophan, for example ATCC no. 44076 or PEP4-1 (Jones, Genetics 85: 12 [1977]). The presence of the trpl lesion as a characteristic of the yeast host cell genome then provides an effective means of selection by growth in the absence of tryptophan.

Suitable promoting sequences for use with yeast hosts include the promoters for 3-phosphoglycerate kinase (Hitzeman et al., "J. Biol. Chem." 255: 2073 [1980]) or other glycolytic enzymes (Hess et al., "J. Adv. Enzyme Reg." 7: 149 [1968]; and Holland, "Biochemistry" 17: 4900 [1978]), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated iwht nitrogen metabolism, metallothionein, glyceraldehyde-3-phosphate dehydrogenase, and enzymes responsible for maltose and galactose utilization. Suitable vectors and promoters for use in yeast expression are further described in R. Hitzeman et al., European Patent Publication No. 73,657A. Yeast enhancers also are advantageously used with yeast promoters.

Preferred promoters controlling transcription from vectors in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. beta actin promoter. The early and late promoters of the SV40 virus are conveniently obtained as an SV40 restriction fragment which also contains the SV40 viral origin of replication. Fiers et al., Nature, 273: 113 (1978). The immediate early promoter of the human cytomegalovirus is conveniently obtained as a HindIII E restriction fragment. Greenaway, P.J. et al., Gene 18: 355-360 (1982). Of course, promoters from the host cell or related species also are useful herein.

Transcription of a DNA encoding tissue factor protein by higher eukaryotes is increased by inserting an enhancer sequence into the vector. Enhancers are cis-acting elements of DNA, usually from about 10 to 300bp, that act on a promoter to increase its transcription initiation capability. Enhancers are relatively orientation and position independent having been found 5' (Laimins, L. et al., Proc. Natl. Acad. Sci. 78: 993 [1981]) and 3' (Lusky, M.L., etal., Mol. Cell Bio. 3: 1108 [1983]) to the transcription unit, within an intron (Banerji, J.L. et al., Cell 33: 729 [1983]) as well as within the coding sequence itself (Osborne, T.F., et al., Mol. Cell Bio. 4: 1293 [1984]). Many enhancer sequences are now known from mammalian genes (globin, elastase, albumin, α-fetoprotein and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers.

Expression vectors used in eukaryotic host cells (yeast, fungi, insect, plant, animal, human or nucleated cells) may also contain sequences necessary for the termination of transcription which may affect mRNA expression. These regions are transcribed as polyadenylated segments in the untranslated portion of the mRNA encoding tissue factor protein. The 3' untranslated regions also include transcription termination sites.

Expression vectors may contain a selection gene, also termed a selectable marker. Examples of suitable selectable markers for mammalian cells are dihydrofolate reductase (DHFR), thymidine kinase or neomycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are: CHO DHFR⁻ cells and mouse LTK⁻ cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non supplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, Southern P. and Berg, P., J. Molec. Appl. Genet. 1: 327 (1982), mycophenolic acid, Mulligan, R.C. and Berg, P. Science 209: 1422 (1980) or hygromycin, Sugden, B. et al., Mol. Cell. Biol. 5: 410-413 (1985). The three examples given above employ bacterial genes under eukaryotic control to convey resistance to the appropriate drug G418 or neomycin (geneticin), xgpt (mycophenolic acid) or hygromycin, respectively.

"Amplification" refers to the increase or replication of an isolated region within a cell's chromosomal DNA. Amplification is achieved using a selection agent e.g. methotrexate (MTX) which inactivates DHFR. Amplification or the making of successive copies of the DHFR gene results in greater amounts of DHFR being produced in the face of greater amounts of MTX. Amplification pressure is applied notwithstanding the presence of endogenous DHFR, by adding ever greater amounts of MTX to the media. Amplification of a desired gene can be achieved by cotransfecting a mammalian host cell with a plasmid having a DNA encoding a desired protein and the HDFR or amplification gene permitting cointegration. One ensures that the cell requires more DHFR, which requirement is met by replication of the selection gene, by selecting only for cells that can grow in the presence of ever-greater MTX concentration. So long as the gene encoding a desired heterologous protein has cointegrated with the selection gene replication of this gene gives rise to replication of the gene encoding the desired protein. The result is that increased copies of the gene, i.e. an amplified gene, encoding the desired heterologous protein express more of the desired heterologous protein.

Preferred suitable host cells for expressing the vectors of this invention encoding tissue factor protein in higher eukaryotes include: monkey kidney CV1 line transformed by SV40 (COS-7, ATCC CRL 1651); human embryonic kidney line (293, Graham, F.L. et al. J. Gen Virol. 36: 59 [1977]); baby hamster kidney cells (BHK, ATCC CCL 10); chinese hamster ovary-cells-DHFR (CHO, Urlaub and Chasin, Proc.Natl.Acad.Sci. (USA) 77: 4216, [1980]); mouse sertoli cells (TM4, Mather, J.P., Biol. Reprod. 23: 243-251 [1980]); monkey kidney cells (CV1 ATCC CCL 70); african green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); and, TRI cells (Mather, J.P. et al., Annals N.Y. Acad. Sci. 383: 44-68 [1982]).

"Transformation" means introducing DNA into an organism so that the DNA is replicable, either as an extrachromosomal element or by chromosomal integration. Unless indicated otherwise, the method used herein for transformation of the host cells is the method of Graham, F. and van der Eb, A., Virology 52: 456-457 (1973). However, other methods for introducing DNA into cells such as by nuclear injection or by protoplast fusion may also be used. If prokaryotic cells or cells which contain substantial cell wall constructions are used, the preferred method of transfection is calcium treatment using calcium chloride as described by Cohen, F.N. et al., Proc. Natl. Acad. Sci. (USA), 69: 2110 (1972).

Construction of suitable vectors containing the desired coding and control sequences employ standard ligation techniques. Isolated plasmids or DNA fragments are cleaved, tailored, and religated in the form desired to form the plasmids required.

For analysis to confirm correct sequences in plasmids constructed, the ligation mixtures are used to transform E. coli K12 strain 294 (ATCC 31446) and successful transformants selected by ampicillin or tetracycline resistance where appropriate. Plasmids from the transformants are prepared, analyzed by restriction and/or sequenced by the method of Messing et al., Nucleic Acids Res. 9: 309 (1981) or by the method of Maxam et al., Methods in Enzymology 65: 499 (1980).

Host cells can be transformed with the expression vectors of this invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily skilled artisan.

"Transfection" refers to the taking up of an expression vector by a host cell whether or not any coding sequences are in fact expressed. Numerous methods of transfection are known to the ordinarily skilled artisan, for example, CaPO₄ and electroporation. Successful transfection is generally recognized when any indication of the operation of this vector occurs within the host cell.

In order to facilitate understanding of the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences in the DNA. The various restriction enzymes used herein are commercially available and their reaction conditions, cofactors and other requirements were used as would be known to the ordinarily skilled artisan. For analytical purposes, typically 1 µg of plasmid or DNA fragment is used with about 2 units of enzyme in about 20 µl of buffer solution. For the purpose of isolating DNA fragments for plasmid construction, typically 5 to 50 µg of DNA are digested with 20 to 250 units of enzyme in a larger volume. Appropriate buffers and substrate amounts for particular restriction enzymes are specified by the manufacturer. Incubation times of about 1 hour at 37°C are ordinarily used, but may vary in accordance with the supplier's instructions. After digestion the reaction is electrophoresed directly on a polyacrylamide gel to isolate the desired fragment.

"Recovery" or "isolation" of a given fragment of DNA from a restriction digest means separation of the digest on polyacrylamide or agarose gel by electrophoresis, identification of the fragment of interest by comparison of its mobility versus that of marker DNA fragments of known molecular weight, removal of the gel section containing the desired fragment, and separation of the gel from DNA. This procedure is known generally (Lawn, R. et al., Nucleic Acids Res. 9: 6103-6114 [1981], and Goeddel, D. et al., Nucleic Acids Res. 8: 4057 [1980]).

"Dephosphorylation" refers to the removal of the terminal 5' phosphates by treatment with bacterial alkaline phosphatase (BAP). This procedure prevents the two restriction cleaved ends of a DNA fragment from "circularizing" or forming a closed loop that would impede insertion of another DNA fragment at the restriction site. Procedures and reagents for dephosphorylation are conventional (Maniatis, T. et al., Molecular Cloning, 133-134 Cold Spring Harbor, [1982]). Reactions using BAP are carried out in 50mM Tris at 68°C to suppress the activity of any exonucleases which may be present in the enzyme preparations. Reactions were run for 1 hour. Following the reaction the DNA fragment is gel purified.

"Ligation" refers to the process of forming phosphodiester bonds between two double stranded nucleic acid fragments (Maniatis, T. et al., Id. at 146). Unless otherwise provided, ligation may be accomplished using known buffers and conditions with 10 units of T4 DNA ligase ("ligase") per 0.5 µg of approximately equimolar amounts of the DNA fragments to be ligated.

"Filling" or "blunting" refers to the procedures by which the single stranded end in the cohesive terminus of a restriction enzyme-cleaved nucleic acid is converted to a double strand. This eliminates the cohesive terminus and forms a blunt end. This process is a versatile tool for converting a restriction cut end that may be cohesive with the ends created by only one or a few other restriction enzymes into a terminus compatible with any blunt-cutting restriction endonuclease or other filled cohesive terminus. Typically, blunting is accomplished by incubating 2-15µg of the target DNA in 10 mM MgCl₂, 1mM dithiothreitol, 50mM NaCl, 10mM Tris (pH 7.5) buffer at about 37°C in the presence of 8 units of the Klenow fragment of DNA polymerase I and 250 µM of each of the four deoxynucleoside triphosphates. The incubation generally is terminated after 30 min. phenol and chloroform extraction and ethanol precipitation.

Human tissue factor protein and its recombinant expression product is obtained according to the following protocol:
1. Oligonucleotide probes representing a single codon choice for each amino acid corresponding to the amino terminal portion of tissue factor protein, and the CNBr peptide fragment were chemically synthesized.
2. Two deoxyoligonucleotides complementary to codons for amino acid sequences of tissue factor protein, described below, were synthesized and radiolabelled with γ³² P-ATP. 
a) 5' CTG ACC TGG AAG TCC ACC AAC TTC AAG ACC ATC CTG-
GAG TGG GAG CCC AAG CCT GTG AAC -3';
 and 
b) 5' ATG GGC CAG GAG AAG GGC GAG TTC CGG GAG ATC TTC-
TAC ATC ATT GGC GCT GTG GTC TTT GTG GTG ATC ATC-
CTG GTG ATC -3'.
3. Oligo (dT) primed cDNA libraries were constructed in λgt10.
4. A human placental cDNA library was screened using the chemically synthesized oligonucleotide probes. No positive plaques were obtained using the 60 mer probe (a). Twenty-two (22) positive plaques were obtained using the 81 mer probe (b), half of which were very weakly positive. The eleven (11) best were chosen to rescreen for plaque purification. Five positive plaques were obtained on the second screen. DNA was prepared from each of these.
5. Clones having a total cDNA of approximately 2800 bp of insert DNA were isolated. Sequencing and characterization of the placental clones were undertaken. Since the mRNA size on a Northern blot was approximately 2.35 Kb these clones may have contained unexcised introns. Hence a human adipose library was screened.
6. An oligo (dT) primed human adipose library was screened using a 1400 bp EcoRI fragment from one of the placental clones.
7. Clones having a total cDNA of approximately 2350 bp (including 150 to 200 bp for the polyA tail) and 1800 bp of insert DNA were isolated. Those clones containing 2350 bp and presumed to contain all the tissue factor mRNA were sequenced.
8. The full length cDNA encoding human tissue factor protein is constructed in a plasmid. It should be appreciated that knowledge of the complete DNA sequence in Fig. 2 enables one to prepare extremely long probes having perfect homology with human tissue factor protein cDNA, thereby considerably simplifying and increasing the efficiency of probing cDNA or genomic libraries from other species, and making it possible to dispense with tissue factor protein purification, sequencing, and the preparation of probe pools.
9. The cDNA encoding human tissue factor protein is then constructed into an expression vehicle which is used to transform an appropriate host cell, which is then grown in a culture to produce the desired tissue factor protein.
10. Biologically active tissue factor protein is produced according to the foregoing procedure has 263 amino acids.

The following examples merely illustrate the best mode now contemplated for practicing the invention, but should not be construed to limit the invention.

### EXAMPLE 1

### cDNA Cloning

DNA encoding tissue factor protein may be obtained by chemical synthesis when the complete DNA sequence is known, by screening reverse transcripts of mRNA from various tissues, or by screening genomic libraries from any cell. Since neither the complete amino acid nor DNA sequence of tissue factor protein were known at the time of this invention, the chemical synthesis of the complete DNA sequence encoding tissue factor protein was not possible.

A human placental cDNA library was prepared as previously described (Ullrich, A. et al., Nature 309:418-425 [1984]). Double-stranded cDNA was prepared from human adipose RNA using reverse transcriptase in known fashion and, after E. coli RNase H treatment DNA polymerase I was used to synthesize the second strand and then ligated to synthetic oligonucleotides containing restriction sites for SalI, XhoI and an EcoRI overhanging end, as described previously (Gubler, U. and Hoffman, B.J., Gene 25: 263 [1983]). This DNA was inserted into the EcoRI site of λgt10 (Huynh, T. et al., DNA Cloning Techniques [ed. Grover, D. ][1984]).

Two oligonucleotide probes representing one possible codon choice for each amino acid of the N-terminal amino acid sequence (60 nucleotides) and the internal amino acid sequence near the C-terminal (81 nucleotides) were designed and synthesized based on the following amino acid sequences presented at an American Heart Association meeting as cited above: 
Amino terminal

5' CTG ACC TGG AAG TCC ACC AAC TTC AAG ACC ATC CTG GAG-
Leu Thr Trp Lys Ser Thr Asn Phe Lys Thr Ile Leu Glu-

TGG GAG CCC AAG CCT GTG AAC -3'
Trp Glu Pro Lys Pro Val Asn
 
Near C-terminal

5' ATG GGC CAG GAG AAG GGC GAG TTC CGG GAG ATC TTC TAC-
Met Gly Gln Glu Lys Gly Glu Phe Arg Glu Ile Phe Tyr-

ATC ATT GGC GCT GTG GTC TTT GTG GTG ATC ATC CTG GTG ATC-3'
Ile Ile Gly Ala Val Val Phe Val Val Ile Ile Leu Val Ile-
 cDNA clones of human tissue factor protein were obtained using the DNA probes first to screen a human placental cDNA library. A 1400 bp EcoRI fragment from a placental clone was used to screen a human adipose cDNA library.

About 1 million phage from the oligo(dT) primed human placenta cDNA library in λgt10 were grown on twenty-five (25) 15-cm petri plates from which triplicate nitrocellulose filters were lifted. The filters were hybridized with each of the ³²P-end labelled oligonucleotide probes in 0.75M NaCl, 75mM trisodium citrate, 50 mM sodium phosphate (pH 6.8), 5X Denhardt's solution, 20 percent formamide, 10 percent dextran sulfate and 0.2 g/l boiled, sonicated salmon sperm DNA at 42°C overnight and washed for 2 hrs in 0.30M NaCl, 30mM trisodium citrate, 0.1 percent NaDodSO₄, at 42°C. Twenty-two (22) hybridizing duplicate positives were observed with filters hybridized with the tissue factor protein near C-terminal probe. The eleven (11) best were chosen for plaque purification. Tissue factor protein amino terminal probe failed to hybridize. Five clones were positive upon plaque purification. DNA was prepared from each of these and then analyzed by digestion with EcoRI. One clone was shorter and appeared to be a partial clone. Four clones which were identical based on an EcoRI digest were the best candidates for full-length cDNA clones. EcoRI fragments from three of the clones, the partial clone and two of the putative full length clones, were subcloned into M13 phage vectors for DNA sequencing by dideoxy chain termination (Messing, J. et al., Nucleic Acids Res. 9:309-321 [1981]).

A 1400 bp EcoRI fragment from placental cDNA clone was hybridized to a Northern blot to which was bound mRNA. The size of the tissue factor protein mRNA was determined to be about 2.35 kb in the placental samples which tested positively. The placental cDNA clones were approximately 2800 bp in length including the nucleotides corresponding to the polyA tail on the mRNA. These clones were approximately 450 bp longer than the observed length of the mRNA on the Northern blot. Stop codons and methionine codons in all three reading frames were observed immediately upstream of the DNA encoding the amino terminus of the protein, suggesting the absence of a signal sequence. The lack of a signal sequence immediately 5' of the sequence representing the NH₂ terminus of the mature protein in the placental clones was confirmed by comparison to the adipose clones described below. It was also determined by comparison of the placental and adipose sequences that the placental clones contained an intervening sequence or intron not present in the adipose clone. The presence of the intron in the placental clone suggests a poor splicing mechanism in the placenta making the isolation and cloning of the pre-tissue factor protein DNA a most difficult task.

Because of the discrepancy in length between the isolated placental clones and the mRNA as determined in Northern blotting tissue factor protein cDNA was also isolated from an adipose library. An adipose cDNA library constructed in λgt10 was chosen because adipose tissue has amounts of tissue factor mRNA comparable to placental tissue. The library was screened using a 1400 bp EcoRI fragment from a placental clone radiolabelled with γ³²-P-ATP under conditions more stringent than those used to screen the placental library. (The above conditions were modified to use 50% formamide in the hybridization; and the wash in 0.03M NaCl, 3mM trisodium citrate, 0.1 percent NaDodSO₄, at 60°C.) Fourteen double positives of varying intensities were obtained. Twelve were chosen for plaque purification. Upon rescreening for plaque purification, 8 strong double positives were obtained. DNA was prepared from each of these positives. Four of these, which were identical upon digestion with EcoRI, were the best candidates for full length cDNA clones. One of these was chosen for analysis by DNA sequencing and labeled λTF14. The size of these clones was approximately 2350 bp, including the length of the polyA tail. This was the same size as observed on Northern blot as described above. A fifth clone was shorter than the 2350 bp clones described above.

Two of the adipose cDNA clones were shorter than the full length mRNA (approximately 1800 bp) and had EcoRI digestion patterns which were distinctly different from the putative full length clones. Analysis of these clones indicates that they are partial clones in that they include DNA corresponding to a portion of the tissue factor protein mRNA. The eighth clone was only about 850 bp and was not chosen for further analysis.

### EXAMPLE 2

### DNA Sequence of Tissue Factor Protein cDNA

The nucleotide sequence of tissue factor protein cDNA is shown in Fig. 2. Of the four adipose clones having an identical EcoRI digestion pattern, one was fully sequenced and corresponded to the sequence shown in Figure 2. Clone λTF14 contains about 2217 bp of insert, which includes approximately 90 nucleotides of the poly(A) tail (which is not shown in Fig 2). The cDNA sequence contains 99 bp of 5' untranslated sequence. The EcoRI digestion pattern of the putative full length clone comprised three fragments of about 900, 750 and 650 bp. A fifth clone appeared to differ in the EcoRI digestion pattern in the fragment at the 5' end. Two of the adipose clones had an EcoRI digestion pattern indicating they were shorter than the full length clones but yet contained an EcoRI fragment longer than any fragment in the full length clones. This may be due to an EcoRI polymorphism or to the presence of an intron. The longest clone was sequenced to completion. Completeness of the coding sequence was assessed from the presence of a long open reading frame beginning with a start codon, ATG. Following the ATG initiator codon are codons for a hydrophobic leader or signal sequence.

The 5' end of the cDNA contains an ATG start codon, for the amino acid methionine, followed by a continuous open reading frame that codes for a 295 amino acid polypeptide. The first 32 amino acid residues are mostly hydrophobic amino acids and probably represent an amino-terminal signal peptide. The amino-terminal sequence that follows corresponds to that sequence of tissue factor protein as purified from tissue. The cDNA sequence predicts that the mature tissue factor protein contains 263 amino acids with a calculated molecular weight of about 29,500. Tissue factor protein is known to be a membrane glycoprotein with a relative molecular mass of 42,000 to 53, 000 on SDS polyacrylamide gels. The translated DNA sequence predicts four (4) asparagine linked glycosylation sites. A hydropathy profile of the protein (Fig. 5) reveals that the first three of these sites are located in hydrophilic regions, increasing the likelihood that they are on the surface of the protein and indeed glycosylated. Likewise, a cluster of 7 out of 13 residues (amino acids 160-172) that are either serine or threonine, indicating possible sites of 0-linked glycosylation, also lies in a predicted region of hydrophilicity. The hydropathy profile also reveals a striking cluster of hydrophobic residues near the carboxy terminus (Fig. 5). This region, encompassing amino acids 220-243, probably comprises the membrane anchoring domain of tissue factor. A search of the sequence data bases revealed no significant homology of tissue factor to available protein sequences. Notably, there was no marked homology to factor VIII, a protein cofactor of the coagulation protease factor IX. This is unexpected because both the factor VIII-factor IX, and the tissue factor-factor VII complexes catalyze the activation of factor X, and the proteases of each complex (factor IX and VII) are highly homologous (F.S. Hagen et al., Proc. Natl. Acad. Sci. USA 83:2412 [1986] and S. Yoshitake et al., Biochemistry 24: 3736 [1985]). It can now be seen that these interactions are not reflected in a similarity of primary protein sequence of the two cofactors.

The cDNA sequence implies that mature tissue factor is released by signal peptidase cleavage of a prepeptide without additional propeptide processing. The 32 amino acids from the initial methionine to the mature amino terminus commence with a charged region followed by a hydrophobic "core" sequence of 14 residues. The prepeptide ends in ala-gly-ala; ala-X-ala is the most frequent sequence preceding signal peptidase cleavage (O. Perlman et al., Mol. Biol. 167:391 [1983]).

The methionine codon at nucleotide 100-102 (Figure 2) is presumed to initiate translation of pre-tissue factor protein. The five nucleotides preceding and the one following this ATG are common choices for nucleotides surrounding translation initiation sites in eukaryotic mRNA, although they are not in complete identity with the concensus described by Kozak, M., Nucl. Acids Res. 12:857 [1984]. The characterized cDNA clones appear to contain virtually the entire 5' untranslated region of the message.

The cDNA contains a 1139 nucleotide 3' untranslated region in which the common polyadenylation signal AATAAA precedes the poly(A) tail by 23 nucleotides. A noteworthy feature of the untranslated region is the presence of a 300 bp Alu family repeat sequence. There are about 300,000 copies of the Alu repeat in the human genome, and numerous examples of their presence in the introns of genes, where they are removed by splicing during the maturation of mRNA (C.W. Schmid et al., Science 216:1065 [1982] and P.A. Sharp, Nature 301:471 [1983]). Although cytoplasmic poly(A)⁺ mRNA also contains Alu sequences, there have only been two previous specific reports of Alu-like sequences in the 3' untranslated sequence of mRNAs: in the class 1 histocompatibility antigens of mouse and rat, and in the human low density lipoprotein receptor (L. Hood et al., Ann. Rev. Immunol, 1:529 [1983]; B. Majello et al., Nature 314:457 [1985] and T. Yamamoto et al., Cell 39:27 [1984]). Alu sequences are often flanked by short direct repeats, as a likely consequence of their insertion into the genome at staggered double-strand nicks. The Alu sequence in the 3' region of tissue factor cDNA is flanked by a direct repeat of 11 nucleotides, as indicated by arrows in Fig. 2.

### EXAMPLE 3

### Expression of Human Tissue Factor Protein Eukaryote Host

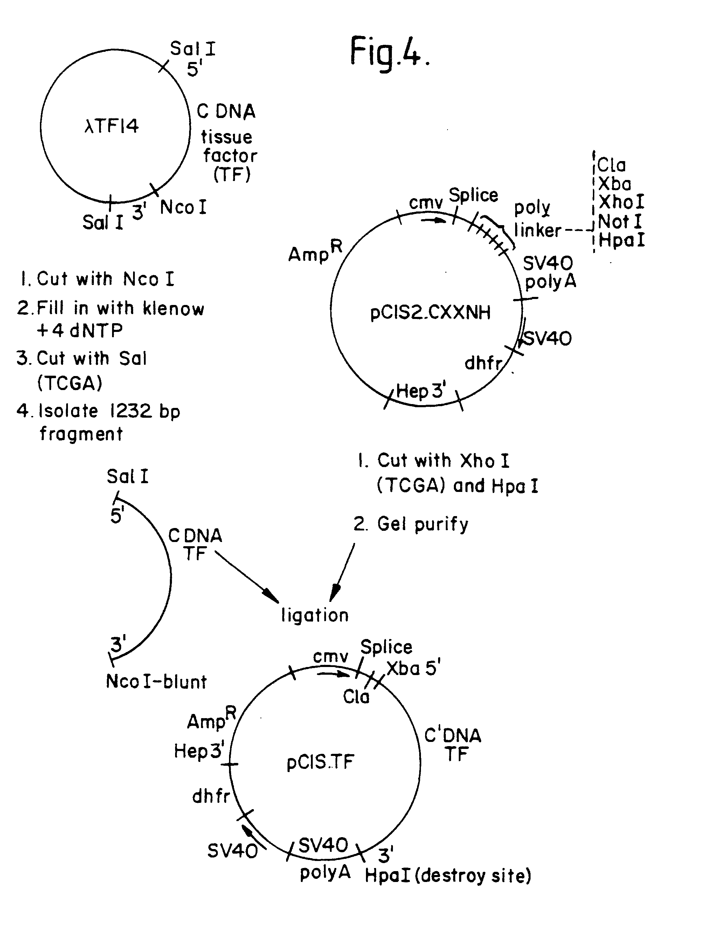

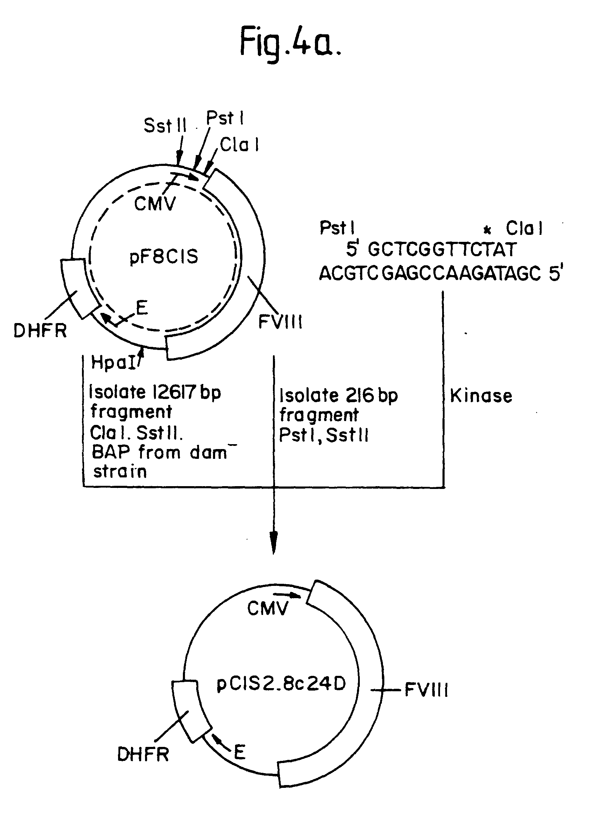

The full length human tissue factor protein cDNA was contained within the cDNA clone λTF14. The full length cDNA was inserted into an expression plasmid comprising the cytomegalovirus enhancer and promoter, the cytomegalovirus splice donor site and intron, the Ig variable region intron and splice acceptor site, the SV40 polyadenylation and transcription termination site. Construction of the expression vector, shown in Figure 4, was undertaken as follows.

The basic vector referred to as pCIS2.8c26D used here is based on pFBCIS described in European Application No. 87308060.0 (U.S. Application No. 07/071,674, filed 9 July 1987 and 06/907, 185, filed 12 Sepember 1986, Docket No. 373P1.

As shown in Figure 4a, a single nucleotide preceding the ClaI site in pFBCIS was changed from guanosine to thymidine so that a dam strain of E. coli would not be required for cutting of the ClaI site.

A three part ligation comprising the following fragments was carried out: a) the 12617bp ClaI-SstII fragment of pFBCIS (isolated from a dam strain and BAP treated); the 216bp SstII-PstI fragment of pFBCIS; and, c) a short Pstl-ClaI synthetic oligonucleotide that was kinased (see Figure 4a, an asterisk indicates the changed nucleotide). This three part ligation generates the expression vector pCIS2.8c24D which is identical to the pCIS2.8c26D and pCIS2,8c28D in the portions used to express tissue factor.

This vector was modified to remove the factor VIII coding sequence by a ClaI-HpaI digest. The region was replaced by a polylinker to allow for additional cloning sites. The sequence of the polylinker used is given below. 
5' CGATTCTAGACTCGAGGTCCGCGGCCGCGTT 3'
3'TAAGATCTGAGCTCCAGGCGCCGGCGCAA 5'
 The ClaI and HpaI sites of the original vector are regenerated and sites for enzymes XbaI, XhoI, NotI were added. This vector is called pCIS2.CXXNH. The coding region for tissue factor was subcloned from λTF14 by using the SalI site present at the 5' junction of the λ vector and the cDNA and a NcoI site located 3' of the coding region in the noncoding portion of the cDNA. A blunt 3' end was first created by digesting with NcoI followed by a fill-in reaction containing the Klenow fragment DNA polymerase and 4 dNTP's. When the λTF14 DNA was subsequently cut with SalI an approximately 1232 bp fragment with the sequence TCGA overhanging at the 5' end and a blunt 3' end containing the tissue factor coding region was created. This was ligated into the pCIS2.CXXNH vector which had been cut with XhoI (yielding a TCGA overhang) and HpaI (blunt). The new vector was labelled pCIS.TF or alternatively referred to as pCISTF1.

Human embryonic kidney cells (293 cells) and monkey kidney cells (Cos cells) were transfected with the expression vector pCIS.TF containing the tissue factor protein cDNA. 48 hours after transfection the cells were harvested and tested for tissue factor protein activity by the chromogenic assay described below Cells were removed from the 100 mm plates by suspension in 1 ml of 0.01 M sodium phosphate buffer, pH 7.0, containing 0.15 M NaCl. The absorbance at 550 nM was adjusted to 0.750 in order to adjust for differential cell density onthe plates. The cells were sonicated for 1 min and Triton X-100 was added to a final concentration of 0.1 percent. The samples were rotated at room temperature for 90 min and cellular debris removed by centrifugation at 10,000 x g. Detergent solubilized extracts were relipidated by diluting 2 µl of the sample into 0.8 ml 0.05 M Tris-HCl, pH 7.5, containing 0.1 M NaCl, 0.1 percent bovine serum albumin (TBS buffer). Fifty µl of a 5 mg/ml solution of phosphotidylcholine (lecithin) in 0.25 percent deoxycholic acid and 25 µl of CdCl₂ were added and the solution incubated for 30 min at 37°C.

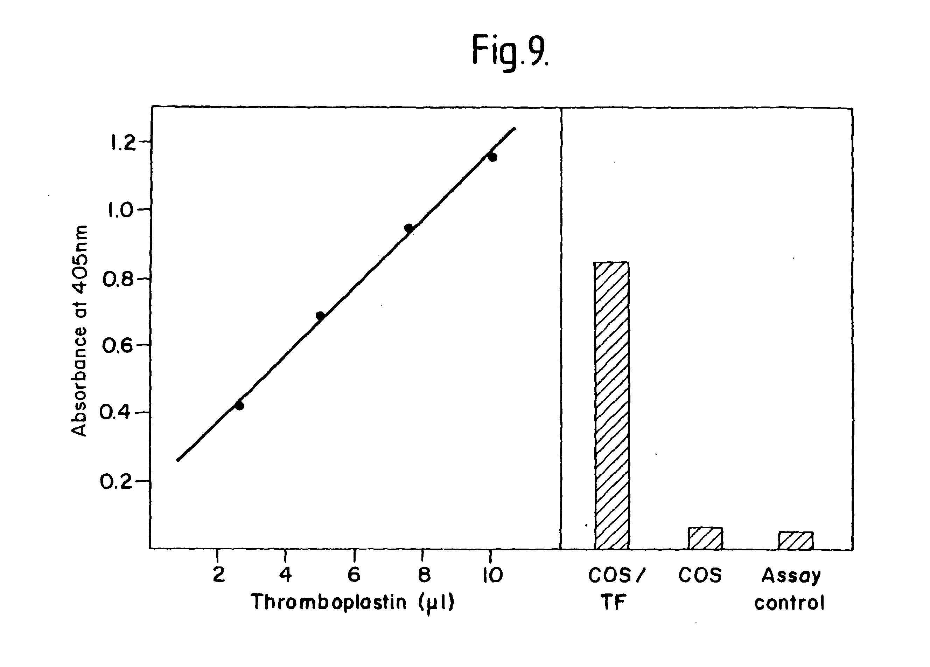

Recombinant tissue factor protein was tested for its ability to function in a specific chromogenic assay. The results are shown in Fig. 9. As expected, various concentrations of rabbit brain thromboplastin (crude tissue factor) were found to react in the assay. Control COS-7 cells (containing the parent expression vector without tissue factor cDNA) had an activity only slightly above the assay blank (with the addition of relipidation mixture alone) (Fig. 9). The cells transfected with the tissue factor expression plasmid, in contrast, showed a strong positive reaction in the assay, thereby demonstrating that the cDNA encodes tissue factor.

### EXAMPLE 4

### Expression of Human Tissue Factor Protein Prokaryote Host

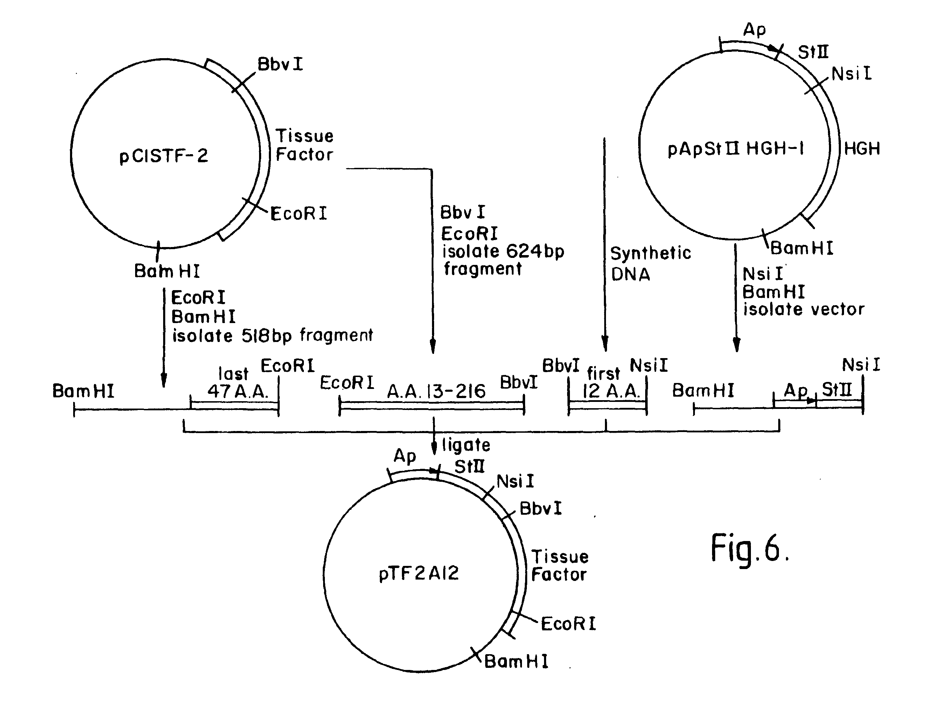

The plasmid pTF2A12 was designed to express mature tissue factor protein in E. coli using the alkaline phosphatase promoter and the STII leader sequence (U.S. Patent 4,680,262). This plasmid was constructed as shown in Figure 6, by the ligation of four DNA fragments, the first of which was the synthetic DNA duplex: 
5'     TCAGGCACTACAAATACTGTGGCAGCATATAATT
  ACGTAGTCCGTGATGTTTATGACACCGTCGTATATTAAATTG-5'
 The above fragment codes for the first 12 amino acids of mature tissue factor protein. The second was a 624 base pair BbvI-EcoRI restriction fragment from pCISTF, described above, coding for amino acids 13 to 216. The third was a 518 base pair EcoRI-BamHI fragment from pCISTF which encodes the last 47 amino acids of tissue factor protein. Plasmid pATSTII HGH-1 (U.S. Patent 4,680,262) was digested with 930 bp NsiI BamHI to produce a fragment.

The four fragments were ligated together to form plasmid pTF2A12, as shown in Figure 6, and used to transform E. coli K12 strain 294 cells. Transformants were selected by ampicillin resistance and plasmid pTF2A12 was selected by restriction analysis and dideoxy sequencing.

E. coli K12 strain 294 cells containing expression vectors were grown overnight at 29°C in low phosphate media containing 50 µg/ml carbenicillin. Cell pellets from 1 ml of culture with an absorbance of 1 at 600 nM were resuspended in 200 µl 50 mM Tris-HCl pH 7.5, 150 mM NaCl, 1% Triton C-100, 1 mg/ml lysozyme. The suspensions were pulse sonicated for 1 minute followed by centrifugation at 10,000 x g to remove cell debris. Detergent solubilized extracts were relipidated by diluting 10 µl of the sample into .8 ml of .05 M Tris-HCl pH 7.5 containing 0.1 M NaCl, 0.1% bovine serum albumin (TBS buffer). Fifty µl of a 5 mg/ml solution of phosphotidyl choline in 0.25% deoxycholic acid and 25 µl of CdCl₂ were added and the solution incubated for 30 minutes at 37°C.

Tissue factor activity was detected by chromogenic assay as described below.

### EXAMPLE 5

### Expression of Human Tissue Factor Protein Mutant

The plasmid pTFIII is designed to express a mature mutant form of tissue factor protein in E. coli. This mutation converts cysteine at position 245 of mature tissue factor protein to serine. The controlling elements for expression, the alkaline phosphatase promoter and STII leader sequence are identical to that used in constructing plasmid pTF2A12.

Plasmid TFIII was made in three steps, the first two of which reconstruct the carboxyl end of the gene. The plasmids pTF100-1 and pTR80-3 are the results of the first two steps.

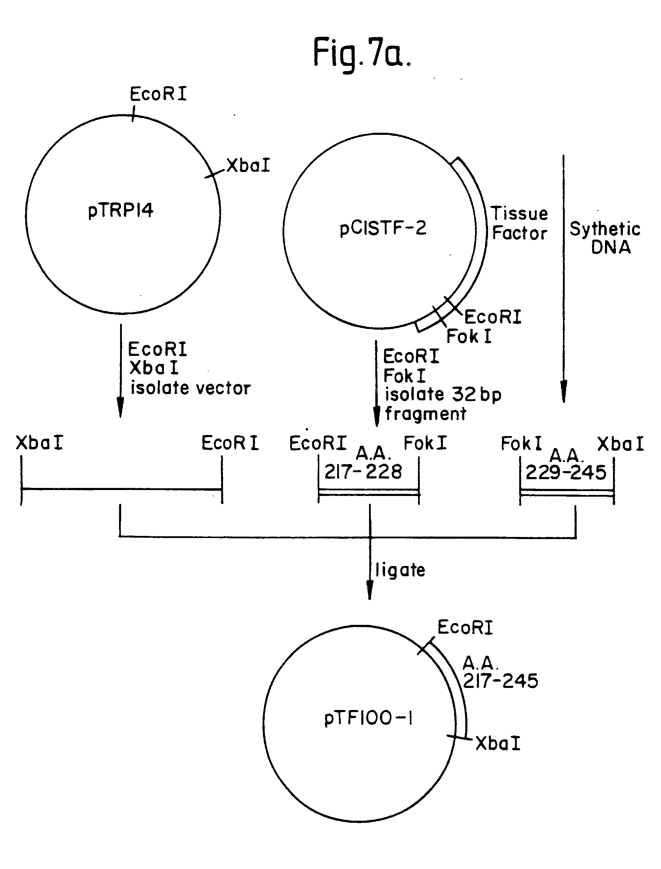

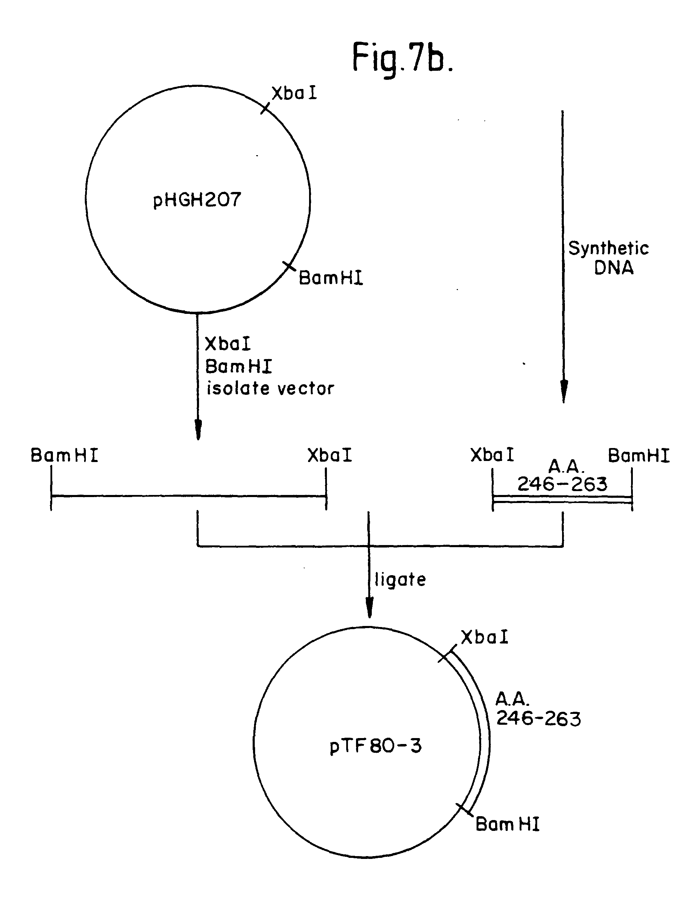

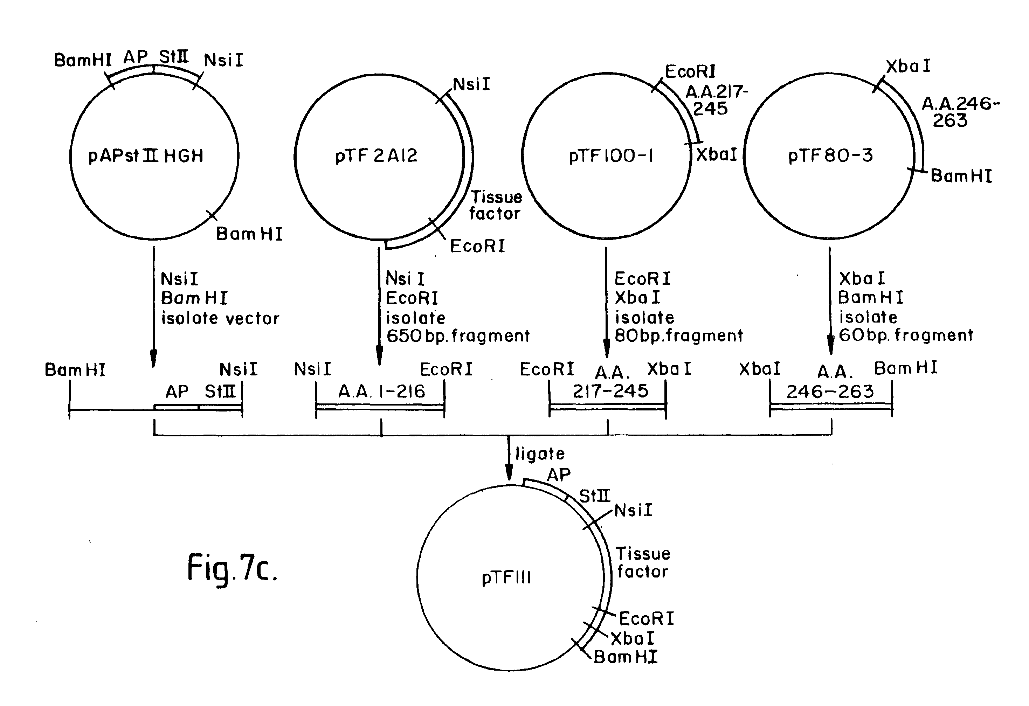

Plasmid pTF100-1 was constructed from three DNA fragments (see Fig. 7a). The first in the cloning vector pTrp14 (U.S. Patent No. 4,663,283) in which a non essential EcoRI-XbaI fragment is removed (Figure 7). The second was a 32 base pair EcoRl-Fokl fragment encoding amino acids 217 to 228 to mature tissue factor protein. The third fragment encoding amino acids 229-245 was a chemically synthesized DNA duplex wherein the codon at position 245 was changed to TCT (underlined) from TGT: 
5'-TA TTT GTG GTC ATC ATC CTT GTC ATC ATC CTG
A CAC CAG TAG TAG GAA CAG TAG TAG GAC

GCT ATA TCT CTA CAC AAG T
CGA TAT AGA GAT GTG TTC AGA TC-5'
 The three fragments were ligated together forming plasmid pTF100-1 and transformed into E. coli K12 strain 294. Transformants were selected by ampicillin resistance and plasmid pTF100-1 was selected by restriction analysis and dideoxy sequencing.

Plasmid pTF80-3 was constructed from two DNA fragments (Figure 7b). The first was plasmid vector pHGH207 (U.S. Patent No. 4,663,283) in which the 930 base pair XbaI-BamHI fragment had been removed. The second was the chemically synthesized 68 mer DNA duplex: 
5'-CTA GAA AGG CAG GAG TTG GCC AGA GCT CGA AGG AGA ACT
TT TCC GTC CTC AAC CGG TCT CGA GCT TCC TCT TGA

CCC CAC TGA ATG TTT CAT AAG CTT G
GGG GTG ACT TAC AAA GTA TTC GAA CCT AG-5'
 The two fragments were ligated together forming plasmid pTF80-3 and transfected into E. coli K12 strain 294. Transformants were selected by ampicillin resistance and plasmid pTF80-3 was selected by restriction analysis and dideoxy sequencing.

Plasmid pTFIII was constructed from four DNA fragments (Figure 7c). The first was the vector pAPSTIIHGH (U. S. Patent No. 4,680,262) in which the 930 base pair Nsil-BamHl fragment had been removed. The second was a 650 base pair Nsil-EcoRi restriction fragment from pTF2A12 (see Example 4 above) encoding amino acids 1 to 217 of mature tissue factor protein. The third was an 80 base pair EcoRI-XbaI fragment from pTF100-1 encoding amino acids 218 to 245 of the mutant mature tissue factor protein. The fourth was a 60 base pair XbaI-BamHI fragment from pTF80-3 encoding the last 18 amino acids. The four fragments were ligated together and transformed into E. coli K12 strain 294. Transformants were selected by ampicillin resistance and plasmid pTFIII selected by restriction analysis.

### EXAMPLE 6

### Expression of a Human Tissue Factor Fusion Protein

A fusion with the herpes-gD signal sequence (EP Publication No. 0139416, published May 2, 1985) and the mature portion of human tissue factor cDNA was constructed using the control elements of vector pRK7. pRK7 and pRK5 (see below) were constructed as follows.

### Construction of pRK5 and pRK7

The starting plasmid was pCIS2.8c28D as described above. The base numbers in paragraphs 1 through 6 refer to pCIS2.8c28D with base one of the first T of the EcoRI site preceding the CMV promoter. The cytomegalovirus early promoter and intron and the SV40 origin and polyA signal were placed on separate plasmids.
1. The cytomegalovirus early promoter was cloned as an EcoRI fragment from pCIS2.8c28D (9999-12-1) into the EcoRI site of pUC118 (Yanish-Perron et al. Gene 33:103 [1985]). Twelve colonies were picked and screened for the orientation in which single stranded DNA made from pUC118 would allow for sequencing from the EcoRI site at 1201 to the EcoRI site at 9999. This clone was named pCMVE/P,
2. Single stranded DNA was made from pCMVE/P in order to insert an SP6 (Green M.R, et al., Cell 32:681-694 [1983]) promoter by site-directed mutagenesis. A synthetic 110 mer which contained the SP6 promoter (See Nucleic Acids Res. 12:7041 [1984] figure 1); sequences from -69 to +5 of SP6 promoter were used along with 18bp fragments on either end of the oligomer corresponding to the CMVE/P sequences. Mutagenesis was done by standard techniques and screened using a labeled 110 mer at high and low stringency. Six potential clones were picked and sequenced. A positive was identified and labelled pCMVE/PSP6.
3. The SP6 promoter was checked and shown to be active, for example, by adding SP6 RNA polymerase and checking for RNA of the appropriate size.
4. A Cla-NotI-Sma adapter was made to be inserted from the ClI site (912) to the SmaI site of pUC118 in pCMVE/P (step 1) and pCMVE/PSP6 (step 2). This adapter was ligated into the CLaI-SmaI site of pUC118 and screened for the correct clones. The linker was sequenced in both and clones were labelled pCMVE/PSP6-L and pCMVE/P-L.
5. pCMVE/PSP6-L was cut with Smal (at linker/pUC118 junction) and HindIII (in pUC118). A HpaI (5573) to HindIII (6136) fragment from pSVORAAΔRI 11, described below, was inserted into SmaI-HindIII of pCMVE/PSP6-L This ligation was screened and a clone was isolated and named pCMVE/PSP6-L-SVORAAΔRI.
   a) The SV40 origin and polyA signal was isolated as Xmnl (5475) - Hindlll (6136) fragment from pCIS2.8c28D and cloned into the HindIII to SmaI sites of pUC119. This was named pSVORAA.
   b) The EcoRI site at 5716 was removed by partial digest with EcoRI and filling in with Klenow. The colonies obtained from self-ligation after fill-in were screened and the correct clone was isolated and named pSVO-RAAΔRI 11. The deleted EcoRI site was checked by sequencing and shown to be correct.
   c) The HpaI (5573) to HindIII (6136) fragment of psVORAAΔ11 11 was isolated and inserted into pCMVE/PSP6-L (see 4 above).
6. pCMVE/PSP6-L-SVOrAAΔRI (step 5) was cut with EcoRI at 9999, blunted and self-ligated. A clone without an EcoRI site was identified and named pRK.
7. pRK was cut with SmaI and BamHI. This was filled in with Klenow and religated. The colonies were screened. A positive was identified and named pRKΔBam/Sma 3.
8. The HindIII site was converted to a HpaI site using a converter. (A converter is a piece of DNA used to change one restriction site to another. In this case one end would be complimentary to a HindIII sticky end and at the other end have a recognition site for HpaI.) A positive was identified and named pRKΔBam/Sma, HIII-HpaI 1.
9. pRKΔBam/Sma, HIII-HpaI 1 was cut with PstI and NotI and a RI-HIII linker and HIII-RI linker were ligated in. Clones for each linker were found. However, it was also determined that too many of the HpaI converters had gone in (two or more converters generate a PvuII site). Therefore, these clones had to be cut with HpaI and self-ligated.
10. RI-HIII clone 3 and HIII-RI clone 5 were cut with HpaI, diluted, and self-ligated. Positives were identified. The RI-III clone was named pRK5. A HIII-RI clone was cut with HpaI, diluted and self-ligated. After screening, a positive was identified and named pRK7.

The vector pRK7 contains the CMV promoter and enhancer and splice donor, the IgG intron and splice acceptor, the SP6 promoter, the SV40 poly A signal and SV40 origin of replication: it has no DHFR gene. Construction of the tissue factor protein expression vector was undertaken as follows: the tissue factor protein cDNA from λTF14 was cloned into the SalI site of pSP64 and labelled pSP64TF (Promega Corporation, 1987). pSP64 containing the tissue factor cDNA was cut with NcoI. An 18 mer converter which was not kinased was ligated to the NcoI end to change the NcoI site to an XbaI site. The sequence of the linkers used were: 
NcoI - XbaI Linker
18 mer
NcoI XbaI
5'CATGGAGTCGTAACTGAT 3'
3' CTCAGCATTGACTAGATC 5'
 pSP64 was then digested with BbvI and a 1030 bp fragment was gel isolated. A Pvull-Bbvl approximately 100 bp DNA duplex which was not kinased, contained the sequences for the first thrombin activation site factor VIII and the first 12 amino acids of mature human tissue factor protein. The sequences of the approximately 100 bp was as follows: 
Tissue Factor/Thrombin Fusion

5' CTG GAG GAC AGT TAT GAA GAT ATT TCA GCA TAC TTG CTG AGT AAA
GAC CTC CTG TCA ATA CTT CTA TAA AGT CGT ATG AAC GAC TCA TTT

AAC AAT GCC ATT GAA CCA AGA TCA GGC ACT ACA AAT ACT GTG GCA
TTG TTA CGG TAA CTT GGT TCT AGT CCG TGA TGT TTA TGA CAC CGT

GCA TAT AAT T 3'
CGT ATA TTA AAT TG
 The PvuII-BbvI fragment was ligated to the approximately 1030 bp fragment. A fragment of approximately 1130 bp was gel isolated. This PvuII-XbaI fragment was then ligated into a pSP64 vector labelled pSP64ThTF. A clone was obtained which was sequenced over the area comprising the synthetic 100 mer. This plasmid was digested with PvuII and Xbal in an attempt to isolate a large amount of the insert. However, the Xbal site was not digested. Therefore, the insert was gel isolated by cutting with Pvull and SalI. The SalI site is in the remaining part of the pSP64 polylinker and located next ot the XbaI site. The second fragment containing the herpes-gD signal sequence plus some 5' untranstated region comprised a 275 bp fragment obtained form the pgD-DHFR (EP Publication 0139417, published May 2, 1985). which is digested with PstI-SacII and a 103 bp SacII-PvuII synthetic fragment having the following sequence: 
SacII-PvuII Synthetic Fragment (HSVgD Leader)

5'-GC AAA TAT GCC TTG GCG GAT GCC TCT CTC AAG ATG GCC GAC
3'-GCG CCG TTT ATA CGG AAC CGC CTA CGG AGA GAG TTC TAC CGG CTG

CCC AAT CGA TTT CGC GGC AAA GAC CTT CCG GTC CTG GAC CAG-3'
GGG TTA GCT AAA GCG CCG TTT CTG GAA GGC CAG GAC CTG GTC-5'
 The third segment was obtained by digesting pRK7 with PstII-SaIII and gel isolating an approximately 4700 bp fragment. The final three thrombin activation site 5' to the cDNA encoding tissue factor protein; b) PstI-PvuII fragment containing the herpes-gD signal sequence; and c) the pRK fragment containing the control elements. The 3 pieces described above and clones were obtained and determined to be correct by restriction enzyme digestion and sequencing.

Human embryonic kidney cells (293S) were transfected with the expression vector containing the tissue factor-herpes-gD fusion protein. Human 293 cells are harvested 15 hours after transient transfection. The culture media is removed and 2 mls of extraction buffer (5 mM Tris HCl); 150 mM NaCl:pH 7.5 [TBS] containing 0.1% Triton X-1 00) are added per 100 mm tissue culture plate. The cells are suspended and rotated (end over end) for 45-60 min. at 4°C. The extract is centrifuged at 8000 X g for 20 min. and then loaded directly onto the monoclonal antibody column (3.5 mg 5B6/ml of CNBr activated Sepharose) at a flow rate of 0.8 ml/min. Preparation of an antibody to herpes-gD is described in EP Publication No. 1,139,416, published May 2, 1985. The antibody column is washed with 10 mls of extraction buffer to return the Absorbance (280 nm) to baseline. The column is then washed with 50 mM Tris HCl; 1 M NaCl; 0.1% Triton X-100, pH 7.5 and eluted with 0.1 M glycine; 150 mM NaCl; 0.1% Triton X-100, pH 2. The pH is adjusted to neutral with 1 M Tris HCl, pH 8.5.

The gD portion of the gD-tissue factor fusion protein was cleaved from the fusion protein using thrombin. 1110 units of thrombin (0.33 mg protein) was covalently attached to 0.5 ml CNBr-activated Sepharose according to manufacturer's instructions. 5000 units of gDTF fusion protein is incubated with approximately 150 µl of thrombin Sepharose for 90 min at 37°C (rotated end over end). The thrombin-Sepharose is then removed by centrifugation.

Tissue factor activity was detected by chromogenic assay as described below.

### EXAMPLE 7

### Expression of Cytoplasmic Domain Deleted Tissue Factor Protein

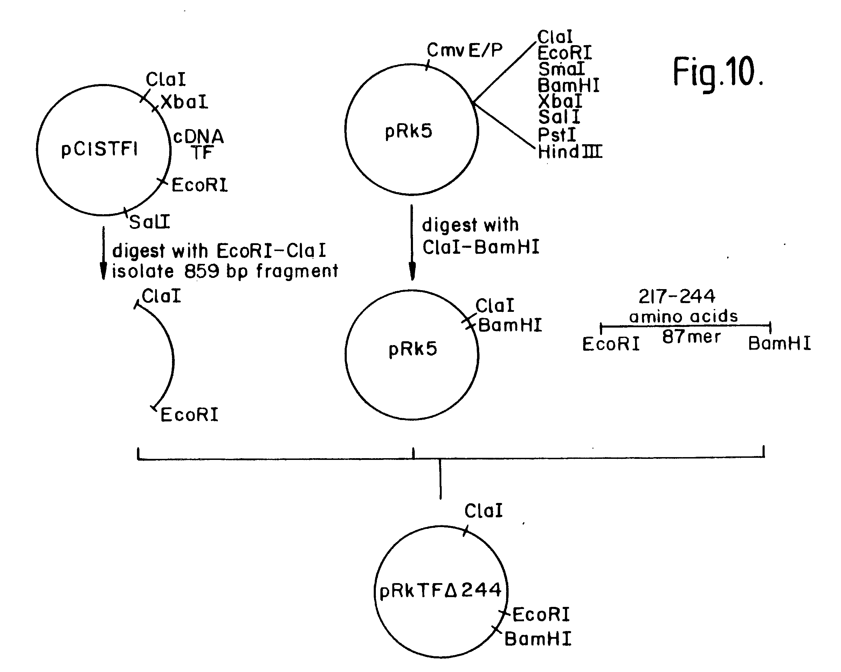

The vector pRKTFΔ244 was constructed, as shown in Figure 10, to express tissue factor protein lacking the cytoplasmic domain, amino acids 244 through 263. The vector was constructed by a three part ligation. The first part was an 859 bp fragment obtained by digesting pCISTF1 with EcoRI and ClaI. The 859 bp was gel isolated. The second portion was gel isolated following Clal-BamHI digestion of pRK5 as described above. The third part was a EcoRI-BamHI chemically synthesized 87 mer having the following sequence:

87 mer

217
Phe Arg Glu Ile Phe Tyr Ile Ile Gly Ala Val Val Phe Val Val
5'-AA TTC AGA GAA ATA TTC TAC ATC ATT GGA GCT GTG GTA TTT GTG GTC
3'-G TCT TTC TAT GAA GTA GAT AAT TCC AGC CAC TAC AAA CAC GAC

243
Ile Ile Leu Val Ile Ile Leu Ala Ile Ser Leu His End
ATC ATC CTT GTC ATC ATC CTG GCT ATA TCT CTA CAC TAA G-3'
GAT GAT AAG GAC GAT GAT CAG AGC TAT AGA TAG GTG TTA TCC GA-5'

The three part ligation used: a) the 859 bp fragment encoding amino acids 1-216; b) the 4700 bp fragment from pRK5; and, c) the 87 mer encoding amino acids 217-243. This new vector was labeled pRKTFΔ244 (see Figure 10).

Human embryonic kidney cells (293) were transfected with the expression vector pRKTFΔ244. After three days, cytoplasmic domain deleted tissue factor protein were purified as previously described and assayed in the chromogenic assay described below. The cytoplasmic-domain deleted tissue factor showed a strong positive reaction in the assay demonstrating that the cytoplasmic domain deleted tissue factor protein was effective in this in vitro coagulation assay.

### EXAMPLE 8

### Purification of Tissue Factor Protein

Tissue factor protein was purified using immunoaffinity purification using an IgG monoclonal antibody that binds human tissue factor protein.

Human tissue factor protein was synthesized in recombinant culture as described above. The following immunogens were injected into a BALB/c mouse (29.1.C) according to the schedule described below: recombinant human tissue factor protein (rTF) (.07 mg/ml having a specific activity 17040 U/mg); recombinant tissue factor protein obtained from a tissue factor-gD fusion cleaved by thrombin to remove the herpes-gD sequences from the amino terminal end (rTF:gDThr) (0.72 mg/ml having a specific activity 4687 U/mg) and recombinant tissue factor-herpes-gD fusion (rTF-gD) (approximately 150,000 U/mg) on the following immunization schedule:

| **Day** | **Administration Route** | **Immunogen** |
|---|---|---|
| 1. | subcutaneous (sc | 0.25 ml of r-TF in Freund's complete adjuvant |
| | | |
| 14. | half sc and half intraperitoneal (ip) | 0.25 ml r-TF:gD in incomplete Freund's adjuvant |
| | | |
| 28. | I.P. | 0.25 ml of r-TF:gD in PBS |
| | | |
| 42. | I.P. | 0.25 ml of r-TF:gD in PBS |
| | | |
| 62. | I.P. | 0.25 ml of r-TF:gD in PBS |
| | | |
| 75. | I.P. | 0.25 ml of r-TF:gD in PBS |
| | | |
| 85. | Intra-Splenic | 0.1 ml of r-TF:gDThr in PBS ** |

| | | |
|---|---|---|
| ** 10-40 µg/ml | | |

The anti-TF titer assayed by radio-immunoprecipitation (RIP) and ELISA increased gradually throughout the immunizations to day 85.

The RIP assay used 0.005 mo of sera from immunized and non-immunized mice diluted with 0.495 ml of PBSTA (PBS containing 0.5% bovine serum albumin [BSA] and 0.1% Triton X-100). 50,000 cpm of ¹²⁵I-rTF was added and the mixture was incubated for 2 hr at room temperature. ¹²⁵I-rTF complexed with antibody was precipitated by incubating for 1 hr at room temperature with 0.05 ml of SPA beads. The SPA beads consisted of staphylococcal protein A bound to sepharose CL-4B beads that had been pre-incubated with rabbit anti-mouse IgG and stored in 50 mM Tris pH 8, 10 mM MgCl₂, 0.1% BSA and 0.02% NaN₃. The beads were pelleted, washed three times with PBSTA and counted in a gamma counter.

The ELISA consisted of 0.1 ml of rTF (0.5 µg/ml) in carbonate buffer pH 9.6 adsorbed to microtiter wells for 2 hr at 37°C. Further non-specific adsorption to the wells was blocked for 1 hr at 37°C with PBSA (PBS containing 5% BSA). The wells were washed 3 times with PBST (PBS containing 0.1% Tween 80) and the serum samples diluted in PBS was added and incubated overnight at 4°C. The wells were washed 3 times with PBST. 0.1 ml of goat anti-mouse immunoglobulin conjugated to horseradish peroxidase was wells were washed again and 0-phenylene diamine was added as substrate and incubated for 25 minutes at room temperature. The reaction was stopped with 2.5 M H₂SO₄ and the absorbance of each well was read at 492 min.

On day 89 the spleen from mouse 29.1.C was harvested, disrupted and the spleen cells fused with P3 X63-Ag8.653 (ATCC CRL 1580) non-secreting mouse myeloma cells using the PEG fusion procedure of S. Fazakas de St. Groth et al., J. Immun. Meth., 35:1-21 (1980). The fused culture was seeded into 4 plates each containing 96 microtiter wells and cultured in HAT (hypoxanthine, aminopterin and thymidine) media by conventional techniques (Mishell and Shiigi, Selected Methods in Cellular Immunology, W.H. Freeman & Co., San Francisco, pp. 357-363 [1980]). The anti-TF activity of culture supernatants was determined by ELISA and RIP. Twenty positives were found to have anti-TF activity. Of these, 12 stable fusions which secreted anti-TF were expanded and cloned by limiting dilution using published procedures (Oi, V.J.T. & Herzenberg, L.A., "Immunoglobulin Producing Hybrid Cell Lines" in Selected Methods in Cellular Immunology, p. 351-372, Mishell, B.B. and Shiigi, S.M. [eds.], W.H. Freeman & Co. [1980]). Selection of clones was based on: macroscopic observation of single clones, ELISA and RIP. The antibody was isotyped using a Zymed isotyping kit according to the accompanying protocol. (Zymed Corp.) Large quantities of specific monoclonal antibodies were produced by injection of cloned hybridoma cells in pristane primed mice to produce ascitic tumors. Ascites were then collected and purified over a protein-A Sepharose column.

Approximately 5 ml of ascites fluid was centrifuged at 3000 rpm in a Sorvall 6000 at 4°C for 10 min. The clear layer of pristane and the layer of lipid was removed with a pasteur pipet. The ascites fluid was transferred to a 50 ml centrifuge tube. The ascites fluid was sterile filtered through a 0.45 M filter. 1.11 gram of KCI was added to the ascites to yield a final concentration of 3M KCI.

The ascites was loaded onto a 10 ml column containing SPA Sepharose (Fermentech). The column was washed with 3M KCI. The mouse IgG was eluted with 3 to 4 column volumes of 0.1 M acetic acid in 0.15 M NaCl pH 2.8.

The antibody D3 was coupled to CNBr Sepharose according to the manufacturer's instructions at 3 mg IgG per ml of Sepharose. (See Pharmacia Co. instruction manual). Transfected 293S cells were grown in a 1:1 mixture of Ham's F-12 (w/o glycine, hypoxanthine and thymidine) and DMEM (w/o glycine). Additions to the basal medium include: 10% dialysed or diafiltered fetal calf serum, 50 nM methotrexate, 2.0 mM L-glutamine and 10 mM HEPES buffer.

A frozen vial of 293S (63/2S CISTF) is thawed in a tissue culture flask containing the described medium. When the culture reaches confluency it is trypsinized with trypsin-EDTA mixture and a small portion of the cell population was used to inoculate larger flasks. Cultures were monitored daily by phase microscopy to determine growth (percent confluency), morphology and general health. When rollerbottle cultures were confluent (usually within 5-7 days), the cells were trypsinized and counted. Cells were enumerated and their viabilities determined by the trypan blue exclusion technique. Typical cell numbers from a confluent 850 cm² rollerbottle were between 1 to 4 x 10⁸ cells. Cells were suspended in 0.01 M sodium phosphate, 0.15 M NaCl. Cells were collected by centrifugation at 5000 rpm. Cells were resuspended in 50 mls TBS containing 1% Triton X per flask. Cultures were incubated one hour at room temperature and then centrifuged 8000 x g for 20 min. Supernatant was loaded onto the D3-Sepharose column described above. The column was washed and eluted with .1 M acetic acid, 150 mM NaCl and .05% Tween 80.

### EXAMPLE 9

### Assay for Tissue Factor Protein

### 1. Chromogenic tissue factor assay.

All samples extracted from the culture medium were relipidated prior to assay. As discussed above tissue factor has an absolute requirement for phospholipid to exhibit activity in in vitro assay systems (Pitlick and Nemerson, Supra). Lecithin granules were homogenized in Tris 0.05 M, 0.1 M NaCl pH7.4 (TBS) containing 0.25% sodium deoxycholate to a concentration of 1 mg/ml. This solution (PC/DOC) was used to relipidate tissue factor as follows. Tissue factor protein was diluted into TBS containing 0.1 % bovine serum albumin (TBSA). Fifty microliters were placed in a 12x75mm polystyrene test tube and 50 µl PC/DOC solution was added. Three hundred and fifty (350) microliters TBSA were then added along with 25 µl 100 mM CdCl₂. This relipidation mixture was allowed to incubate at 37°C for 30 min.

For the chromogenic assay, relipidated tissue factor protein samples were diluted in TBSA. Ten microliters were placed in a test tube with 50 µl of the factor IXₐ/factor X reagent and 2 µl of a solution of purified factor VII, 30 units/ml. The tubes were warmed to 37°C and 100 µl 25mM CaCl₂ were added. Samples were incubated for 5 min. at 37°C prior to the addition of 50 µl chromogenic substrate specific for factor Xa, S2222, also containing the synthetic thrombin inhibitor 12581. The reaction was allowed to proceed for 10 min. and was stopped by the addition of 100 µl 50% glacial acetic acid solution. Absorbance was detected at 405 nM. A standard curve was constructed using rabbit brain thromboplastin (commercially available from Sigma, St. Louis, MO. catalogue #TO263) arbitrarily assigning this reagent as having 100 tissue factor units/ml. Dilutions were made from 1:10 to 1:1000. Absorbance was plotted on the abscissa on semilog graph paper with dilution of standard plotted on the ordinate.

### 2. One stage assay for tissue factor activity.

100 µl haemophilic plasma were added to 10 µl of relipidated or lipid free tissue factor protein or TBSA as control in a siliconized glass tube to prevent non-specific activation through the contact phase of coagulation. The reactants were warmed to 37°C and 100 µl 25 mM CaCl₂ were added and clot formation timed (Hvatum, Y and Prydz, H., Thromb. Diath. Haemorrh. 21, 217-222 [1969]).

### EXAMPLE 10

### Efficacy and Lack of Toxicity of Tissue Factor Protein in a Canine Hemophilia Model

Tissue factor protein was infused into hemophilic dogs using the procedure of Giles, A.R. et al., Blood 60, 727-730 (1982).

Lack of tissue factor protein toxicity was first determined in a normal dog on bolus injection of about 50 tissue factor protein U/kg and 100 tissue factor protein U/kg doses. A cuticle bleeding time (CBT) was performed (Giles supra) prior to infusion and 30 min after each injection. Blood was withdrawn and anticoagulated for coagulation assays at various time points during the experiment. In order to demonstrate in vivo lactor VIII bypassing activity of tissue factor protein, experiments were conducted using hembphilic dogs. Fasting animals were anesthetized and a CBT performed prior to any infusion. Tissue factor protein was then administered by bolus injection and CBTs performed at various time points up to 90 min after the infusion. Several doses of tissue factor protein were administered. Blood samples were withdrawn throughout the duration of each experiment and assayed for factor V, prothrombin and partial thromboplastin times. CBTs of greater than 12 min were regarded as grossly abnormal. Those nails were cauterized to prevent excessive blood loss.

An anesthetized normal dog was administered doses of tissue lactor protein representing 100 U/kg of tissue factor protein on relipidation in the chromogenic assay. The CBT in this animal was approximately 3 min prior to any infusion. There was some reduction in WBCT al 5 minutes while it returned to normal at 15 minutes. Factor V levels were normal 30 min after each infusion. The prothrombin and partial thromboplastin times were unchanged at the end of the experiment and the CBTs were within the normal range. Thus the infusion of tissue factor protein was well tolerated in normal dogs and no evidence of disseminated intravascular coagulation was found.

A hemophilic dog with a prolonged CBT characteristic of hemophilia A was administered 100 U/kg of tissue factor protein. The CBT was normalized at 5 minutes and 20 minutes after this infusion. A second experiment using 100 U/kg of tissue factor protein gave normal CGR at 20 minutes and some shortening of CBT at 90 minutes. The procoagulant effect was not maintained 90 min after the infusion as the CBT effect was again prolonged at this time point.

### Pharmaceutical Compositions

The compounds of the present invention can be formulated according to known methods to prepare pharmaceutically useful compositions, whereby the tissue factor protein product hereof is combined in admixture with a pharmaceutically acceptable carrier vehicle. Suitable vehicles and their formulation, inclusive of other human proteins, e.g. human serum albumin, are described for example in Remington's Pharmaceutical Sciences by E.W. Martin, which is hereby incorporated by reference. Such compositions will contain an effective amount of the protein hereof together with a suitable amount of vehicle in order to prepare pharmaceutically acceptable compositions suitable for effective administration to the host. Such compositions should be stable for appropriate periods of time, must be acceptable for administration to humans, and must be readily manufacturable. An example of such a composition would be a solution designed for parenteral administration. Although pharmaceutical solution formulations are provided in liquid form appropriate for immediate use, such parenteral formulations may also be provided in frozen or in lyophilized form. In the former case, the composition must be thawed prior to use. The latter form is often used to enhance the stability of the medicinal agent contained in the composition under a wide variety of storage conditions. Such lyophilized preparations are reconstituted prior to use by the addition of suitable pharmaceutically acceptable diluent(s), such as sterile water or sterile physiological saline solution. The tissue factor protein of this invention is administered to provide a coagulation inducing therapeutic composition for various chronic bleeding disorders, characterized by a tendency toward hemorrhage, both inherited and acquired. Examples of such chronic bleeding disorders are deficiencies of factors VIII, IX, or XI. Examples of acquired disorders include: acquired inhibitors to blood coagulation factors e.g. factor VIII, von Willebrand factor, factors IX, V, XI, XII and XIII: haemostatic disorder as a consequence of liver disease which includes decreased synthesis of coagulation factors and DIC: bleeding tendency associated with acute and chronic renal disease which includes coagulation factor deficiencies and DIC: haemostasis after trauma or surgery; patients with disseminated malignancy which manilests in DIC with increases in lactors VIII, von Willebrand lactor and fibrinogen: and haemostasis during cardiopulmonary surgery and massive blood transfusion.

## Claims

1. A polynucleotide encoding a biologically active tissue factor protein whose sequence is given in Figure 2 or a biologically active variant or fragment of the said tissue factor protein which induces coagulation wherein at least one amino acid has been selectively inserted. deleted or substituted.

2. A polynucleotide according to Claim 1 encoding either a biologically active tissue factor protein variant which induces coagulation or a biologically active fragment of the said tissue factor protein which induces coagulation.

3. A polynucleotide according to Claim 1 or 2 free of introns.

4. A method of producing biologically active tissue factor protein or a biologically active variant or fragment of the said tissue factor protein which induces coagulation, wherein the said tissue factor protein or variant or fragment lacks glycosylation, or has non-mammalian glycosylation comprising the steps of (a) introducing the polynucleotide of Claim 1 into a non-mammalian host, (b) expressing said polynucleotide and (c) isolating said tissue factor protein or variant or fragment thereof from the host.

5. A method according to Claim 4 wherein the non-mammalian host is any one of a prokaryote, a yeast, a fungus, an insect cell, or a plant cell.

6. A method of producing a biologically active variant or fragment of tissue factor protein which induces coagulation comprising the steps of (a) introducing the polynucleotide of Claim 2 into a host, (b) expressing said polynucleotide and (c) isolating said tissue factor protein variant or fragment of said tissue factor protein.

7. A method according to Claim 6 wherein the host is any one of a prokaryote, a yeast, a fungus, an insect cell, a plant cell, an animal cell or a human cell.

8. A method according to Claim 4 or 6 wherein compared to native tissue factor protein, a selected amino acid is replaced such that in the biologically active tissue factor protein variant (a) a hydrophilic residue is replaced by a hydrophobic residue, or (b) a cysteine or proline is replaced by another amino acid or (c) a residue having an electropositive side chain is replaced by an electronegative residue or (d) a residue having a bulky side chain is replaced by a residue having a small side chain or glycine.

9. A biologically active tissue factor protein or a biologically active variant or fragment of said tissue factor protein which induces coagulation obtainable by the method of Claim 4.

10. A biologically active variant or fragment of tissue factor protein which induces coagulation obtainable by the method of Claim 6.

11. A biologically active tissue factor protein capable of being encoded by a polynucleotide as defined in claim 1 or a biologically active variant or fragment of said tissue factor protein which induces coagulation which either lacks glycosylation, or has non-mammalian glycosylation.

12. A biologically active tissue factor protein variant which induces coagulation capable of being encoded by a polynucleotide as defined in claim 1 wherein the transmembrane domain of native tissue factor protein is not present.

13. A biologically active tissue factor protein variant which induces coagulation capable of being encoded by a polynucleotide as defined in claim 1 wherein a selected site which is a potential proteolysis site in native tissue factor protein is resistant to proteolysis.

14. A biologically active tissue factor protein variant according to Claim 13 wherein a potential proteolysis site in native tissue factor protein comprising an arginine is replaced by another amino acid or is not present.

15. A biologically active tissue factor protein variant according to Claim 14 wherein the arginine is replaced by glutamine or histidine.

16. A biologically active human tissue factor protein variant which induces coagulation capable of being encoded by a polynucleotide as defined in claim 1 wherein a cysteine residue of native human tissue factor protein is replaced by another amino acid residue.

17. A biologically active fusion polypeptide capable of being encoded by a polynucleotide as defined in claim 1 comprising tissue factor protein or a variant or fragment thereof which induces coagulation and another polypeplide fused thereto.

18. A biologically active human tissue factor protein variant which induces coagulation capable of being encoded by a polynucleotide as defined in Claim 1 wherein an N-linked or an O-linked glycosylation site of native human tissue factor protein is not present.

19. A biologically active human tissue factor protein variant according to Claim 16 wherein said cysteine residue is cysteine 245 and the other amino acid residue is serine.

20. A nucleotide sequence encoding a biologically active tissue factor protein or variant or fragment as defined in any one of Claims 11 to 17.

21. A recombinant expression vector comprising a nucleotide sequence according to any one of Claims 1 to 3 or 20.

22. A cell transformed with the expression vector of Claim 21.

23. A cell according to Claim 22 which is a mammalian cell.

24. A cell according to Claim 22 which is a prokaryotic cell.

25. A process for producing tissue factor comprising culturing a transformed cell according to any one of Claims 22 to 24.

26. A method for obtaining a polynucleotide encoding a biologically active tissue factor protein comprising the steps of;
(1) constructing an adipose cDNA library in λgt 10;
(2) screening a library of plaques with an extremely long probe, prepared from the complete DNA sequence in Figure 2, by hybridising DNA in said λ plaques with said probe in 0.75M NaCl, 75 mM trisodium citrate, 50 mM sodium phosphate (pH 6.8), 5X Denhardt's solution, 50 percent formamide and 0.2 g/l boiled, sonicated salmon sperm DNA at 42°C overnight and washing in 0.03M NaCl, 3 mM trisodium citrate, 0.1 percent NaDodSO₄, at 60°C;
(3) purifying the hybridizing plaques;
(4) preparing DNA from hybridizing plaques; and
(5) selecting a cDNA of approximately 2350 bp.

## Patentansprüche

1. Polynucleotid mit Kodierung für ein biologisch aktives Gewebefaktorprotein, dessen Sequenz in Fig. 2 angegeben ist, oder eine biologisch aktive Variante oder ein Fragment dieses Gewebefaktorproteins, die bzw. das Koagulation induziert, wobei mindestens eine Aminosäure selektiv insertiert, deletiert oder ersetzt ist.

2. Polynucleotid nach Anspruch 1 mit Kodierung entweder für eine biologisch aktive Gewebefaktorproteinvariante oder ein biologisch aktives Fragment dieses Gewebefaktorproteins, die bzw. das Koagulation induziert.

3. Polynucleotid nach Anspruch 1 oder 2, das von Intronen frei ist.

4. Verfahren zur Herstellung eines biologisch aktiven Gewebefaktorproteins oder einer biologisch aktiven Variante oder eines Fragments dieses Gewebefaktorproteins, die bzw. das Koagulation induziert, wobei das Gewebefaktorprotein oder die Variante oder das Fragment nicht glykosyliert ist oder eine Nicht-Säugetierglykosylierung aufweist, durch (a) Einführen des Polynucleotids von Anspruch 1 in einen Nicht-Säugetierwirt, (b) Expression des Polynucleotids und (c) Isolieren des Gewebefaktorproteins oder der Variante oder des Fragments hiervon aus dem Wirt.

5. Verfahren nach Anspruch 4, wobei es sich bei dem Nicht-Säugetierwirt um einen Prokaryonten, eine Hefe, einen Pilz, eine Insektenzelle oder eine Pflanzenzelle handelt.

6. Verfahren zur Herstellung einer biologisch aktiven Variante oder eines biologisch aktiven Fragments des Gewebefaktorproteins, die bzw. das Koagulation induziert, durch (a) Einführen des Polynucleotids von Anspruch 2 in einen Wirt, (b) Expression des Polynucleotids und (c) Isolieren der Gewebefaktorproteinvariante oder des Fragments des Gewebefaktorproteins.

7. Verfahren nach Anspruch 6, wobei der Wirt aus einem Prokaryonten, einer Hefe, einem Pilz, einer Insektenzelle, einer Pflanzenzelle, einer Tierzelle oder einer menschlichen Zelle besteht.

8. Verfahren nach Anspruch 4 oder 6, wobei im Vergleich zu nativem Gewebefaktorprotein eine ausgewählte Aminosäure derart ersetzt ist, daß in der biologisch aktiven Gewebefaktorproteinvariante (a) ein hydrophiler Rest durch einen hydrophoben Rest oder (b) ein Cystein oder Prolin durch eine andere Aminosäure oder (c) ein Rest mit einer elektropositiven Seitenkette durch einen elektronegativen Rest oder (d) ein Rest mit einer sperrigen Seitenkette durch einen Rest mit kleiner Seitenkette oder durch Glycin ersetzt ist.

9. Biologisch aktives Gewebefaktorprotein oder eine biologisch aktive Variante oder ein biologisch aktives Fragment dieses Gewebefaktorproteins, die bzw. das Koagulation induziert, erhältlich nach dem Verfahren gemäß Anspruch 4.

10. Biologisch aktive(s) Variante oder Fragment von Gewebefaktorprotein, die bzw. das Koagulation induziert, erhältlich nach dem Verfahren gemäß Anspruch 6.

11. Biologisch aktives Gewebefaktorprotein mit der Fähigkeit, durch ein Polynucleotid gemäß Anspruch 1 kodiert zu werden, oder ein(e) biologisch aktive(s) Variante oder Fragment dieses Gewebefaktorproteins, die bzw. das Koagulation induziert, mit fehlender Glykosylierung oder mit Nicht-Säugetierglykosylierung.

12. Biologisch aktive Gewebefaktorproteinvariante, die Koagulation induziert, mit der Fähigkeit, durch ein Polynucleotid gemäß Anspruch 1 kodiert zu werden, wobei die Transmembrandomäne von nativem Gewebefaktorprotein fehlt.

13. Biologisch aktive Gewebefaktorproteinvariante, die Koagulation induziert, mit der Fähigkeit, durch ein Polynucleotid gemäß Anspruch 1 kodiert zu werden, wobei eine in nativem Gewebefaktorprotein eine mögliche Proteolysestelle darstellende ausgewählte Stelle proteolysebeständig ist.

14. Biologisch aktive Gewebefaktorproteinvariante nach Anspruch 13, wobei eine in nativem Gewebefaktorprotein mögliche Proteolysestelle, umfassend ein Arginin, durch eine andere Aminosäure ersetzt ist oder fehlt.

15. Biologisch aktive Gewebefaktorproteinvariante nach Anspruch 14, wobei das Arginin durch Glutamin oder Histidin ersetzt ist.

16. Biologisch aktive Humangewebefaktorproteinvariante, die Koagulation induziert, mit der Fähigkeit, durch ein Polynucleotid gemäß Anspruch 1 kodiert zu werden, wobei ein Cysteinrest von nativem Humangewebefaktorprotein durch einen anderen Aminosäurerest ersetzt ist.

17. Biologisch aktives Fusionspolypeptid mit der Fähigkeit, durch ein Polynucleotid gemäß Anspruch 1 kodiert zu werden, umfassend Gewebefaktorprotein oder eine Variante oder ein Fragment desselben, die bzw. das Koagulation induziert, und ein weiteres damit verschmolzenes Polypeptid.

18. Biologisch aktive Humangewebefaktorproteinvariante, die Koagulation induziert, mit der Fähigkeit, durch ein Polynucleotid gemäß Anspruch 1 kodiert zu werden, wobei eine N-verbundene oder eine O-verbundene Glykosylierungsstelle von nativem Humangewebefaktorprotein fehlt.

19. Biologisch aktive Humangewebefaktorproteinvariante nach Anspruch 16, wobei es sich bei dem Cysteinrest um Cystein 245 handelt und der andere Aminosäurerest aus Serin besteht.

20. Nucleotidsequenz mit Kodierung für ein biologisch aktives Gewebefaktorprotein oder eine Variante oder ein Fragment gemäß der Definition nach einem der Ansprüche 11 bis 17.

21. Rekombinanter Expressionsvektor, umfassend eine Nucleotidsequenz nach einem der Ansprüche 1 bis 3 oder 20.

22. Mit dem Expressionsvektor nach Anspruch 21 transformierte Zelle.

23. Zelle nach Anspruch 22, bei der es sich um eine Säugetierzelle handelt.

24. Zelle nach Anspruch 22, bei der es sich um eine Prokaryontenzelle handelt.

25. Verfahren zur Herstellung von Gewebefaktor durch Züchten einer transformierten Zelle nach einem der Ansprüche 22 bis 24.

26. Verfahren zur Gewinnung eines Polynucleotids mit Kodierung für ein biologisch aktives Gewebefaktorprotein durch
(1) Konstruieren einer Fett(gewebe)-cDNA-Bibliothek in λgt 10;
(2) Screenen einer Plaquebibliothek mit einer aus der vollständigen DNA-Sequenz gemäß Fig. 2 hergestellten extrem langen Sonde durch Hybridisieren der DNA in den λ-Plaques mit der Sonde in 0,75 M NaCl, 75 mM Trinatriumcitrat, 50 mM Natriumphosphat (pH-Wert: 6,8), 5X Denhardtscher Lösung, 50% Formamid und 0,2 g/l gekochter und beschallter Lachssperma-DNA bei 42°C über Nacht und Waschen in 0,03 M NaCl, 3 mM Trinatriumcitrat, 0,1% NaDodSO₄ bei 60°C;
(3) Reinigen der hybridisierenden Plaques;
(4) Herstellen der DNA aus hybridisierenden Plaques und
(5) Auswählen einer cDNA von etwa 2350 bp.

## Revendications

1. Polynucléotide codant pour une protéine du facteur tissulaire biologiquement active dont la séquence est donnée à la figure 2 ou une variante ou un fragment biologiquement actif (active) de ladite protéine du facteur tissulaire qui induit une coagulation, dans lequel au moins un acide aminé a été sélectivement inséré, supprimé ou substitué.

2. Polynucléotide selon la revendication 1, codant pour une variante de protéine du facteur tissulaire biologiquement active qui induit une coagulation ou pour un fragment biologiquement actif de ladite protéine du facteur tissulaire qui induit une coagulation.

3. Polynucléotide selon la revendication 1 ou 2, exempt d'introns.

4. Procédé de production de protéine du facteur tissulaire biologiquement active ou d'une variante ou d'un fragment biologiquement actif (active) de ladite protéine du facteur tissulaire qui induit une coagulation, dans lequel ladite protéine ou variante ou ledit fragment du facteur tissulaire ne présente pas de glycosylation ou bien a une glycosylation non mammifère, comprenant les étapes consistant à (a) introduire le polynucléotide de la revendication 1 dans un hôte non mammifère, (b) exprimer ledit polynucléotide et (c) isoler ladite protéine du facteur tissulaire ou ladite variante ou ledit fragment de celle-ci, à partir de l'hôte.

5. Procédé selon la revendication 4, dans lequel l'hôte non mammifère est l'un quelconque parmi un procaryote, une levure, un champignon, une cellule d'insecte ou une cellule de plante.

6. Procédé de production d'une variante ou d'un fragment biologiquement actif (active) de protéine du facteur tissulaire qui induit une coagulation, comprenant les étapes consistant à (a) introduire le polynucléotide de la revendication 2 dans un hôte, (b) exprimer ledit polynucléotide et (c) isoler ladite variante de protéine du facteur tissulaire ou ledit fragment de ladite protéine du facteur tissulaire.

7. Procédé selon la revendication 6, dans lequel l'hôte est l'un quelconque parmi un procaryote, une levure, un champignon, une cellule d'insecte, une cellule de plante, une cellule animale ou une cellule humaine.

8. Procédé selon la revendication 4 ou 6, dans lequel, par rapport à une protéine native du facteur tissulaire, un acide aminé choisi est remplacé de sorte que dans la variante de protéine du facteur tissulaire biologiquement active (a) un résidu hydrophile est remplacé par un résidu hydrophobe, ou (b) une cystéine ou une proline est remplacée par un autre acide aminé ou (c) un résidu ayant une chaîne latérale électropositive est remplacé par un résidu électronégatif ou (d) un résidu ayant une chaîne latérale encombrante est remplacé par un résidu ayant une petite chaîne latérale ou de la glycine.

9. Protéine du facteur tissulaire biologiquement active ou une variante ou fragment biologiquement actif (active) de ladite protéine du facteur tissulaire qui induit une coagulation pouvant être obtenu(e) par le procédé de la revendication 4.

10. Variante ou fragment biologiquement actif (active) de protéine du facteur tissulaire qui induit une coagulation, pouvant être obtenu(e) par le procédé de la revendication 6.

11. Protéine du facteur tissulaire biologiquement active capable d'être codée par un polynucléotide tel que défini dans la revendication 1, ou variante ou fragment biologiquement actif (active) de ladite protéine de facteur tissulaire qui induit une coagulation, qui ne présente pas de glycosylation ou bien a une glycosylation non mammifère.

12. Variante de protéine du facteur tissulaire biologiquement active qui induit une coagulation, capable d'être codée par un polynucléotide tel que défini dans la revendication 1, dans laquelle le domaine transmembranaire de la protéine native du facteur tissulaire n'est pas présent.

13. Variante de protéine du facteur tissulaire biologiquement active qui induit une coagulation, capable d'être codée par un polynucléotide tel que défini dans la revendication 1, dans laquelle un site choisi qui est un site de protéolyse potentiel dans une protéine native du facteur tissulaire est résistant à une protéolyse.

14. Variante de protéine du facteur tissulaire biologiquement active selon la revendication 13, dans laquelle un site de protéolyse potentiel dans une protéine native du facteur tissulaire comprenant une arginine est remplacé par un autre acide aminé ou bien n'est pas présent.

15. Variante de protéine du facteur tissulaire biologiquement active selon la revendication 14, dans laquelle l'arginine est remplacée par la glutamine ou l'histidine.

16. Variante de protéine du facteur tissulaire humain biologiquement active qui induit une coagulation, capable d'être codée par un polynucléotide tel que défini dans la revendication 1, dans laquelle un résidu cystéine de la protéine native du facteur tissulaire humain est remplacé par un autre résidu d'acide aminé.

17. Polypeptide de fusion biologiquement actif capable d'être codé par un polynucléotide tel que défini dans la revendication 1, comprenant une protéine du facteur tissulaire ou une variante ou un fragment de celle-ci qui induit une coagulation et un autre polypeptide fusionné à celui-ci (celle-ci).

18. Variante de protéine du facteur tissulaire humain biologiquement active qui induit une coagulation, capable d'être codée par un polynucléotide tel que défini dans la revendication 1, dans laquelle un site de glycosylation lié à N ou lié à O de protéine native du facteur tissulaire humain n'est pas présent.

19. Variante de protéine du facteur tissulaire humain biologiquement active selon la revendication 16, dans laquelle ledit résidu cystéine est la cystéine 245 et l'autre résidu d'acide aminé est la sérine.

20. Séquence de nucléotides codant pour une protéine du facteur tissulaire biologiquement active ou une variante ou un fragment tels que définis dans l'une quelconque des revendications 11 à 17.

21. Vecteur d'expression recombinant comprenant une séquence de nucléotides selon l'une quelconque des revendications 1 à 3, ou 20.

22. Cellule transformée avec le vecteur d'expression de la revendication 21.

23. Cellule selon la revendication 22, qui est une cellule de mammifère.

24. Cellule selon la revendication 22, qui est une cellule procaryote.

25. Procédé pour produire du facteur tissulaire, consistant à cultiver une cellule transformée selon l'une quelconque des revendications 22 à 24.

26. Procédé pour obtenir un polynucléotide codant pour une protéine du facteur tissulaire biologiquement active, comprenant les étapes consistant à :
(1) construire une bibliothèque d'ADNc adipeux dans λgt 10 ;
(2) détecter une bibliothèque de plaques avec une sonde extrêmement longue, préparée à partir de la séquence d'ADN complète de la figure 2, en hybridant l'ADN dans lesdites plaques λ avec ladite sonde dans NaCl 0,75 M, citrate de trisodium 75 mM, phosphate de sodium 50 mM (pH 6,8), solution de Denhardt 5X, formamide à 50 % et 0,2 g/l d'ADN de sperme de saumon bouilli, passé aux ultrasons, à 42 °C pendant une nuit et en lavant dans NaCl 0,03 M, citrate de trisodium 3 mM, NaDodSO₄ à 0,1 %, à 60 °C ;
(3) purifier les plaques hybridées ;
(4) préparer de l'ADN à partir des plaques hybridées ; et
(5) choisir un ADNc d'approximativement 2350
